(19) **Europäisches Patentamt / European Patent Office / Office européen des brevets**

(11) **EP 2 030 572 B1**

(12) **EUROPEAN PATENT SPECIFICATION**

(45) Date of publication and mention of the grant of the patent:
**08.06.2016 Bulletin 2016/23**

(21) Application number: **07743632.7**

(22) Date of filing: **18.05.2007**

(51) Int Cl.:
*A61B 5/00* *(2006.01)*     *A61B 8/00* *(2006.01)*

(86) International application number:
**PCT/JP2007/060197**

(87) International publication number:
**WO 2007/138881 (06.12.2007 Gazette 2007/49)**

(54) **ULTRASONOGRAPHIC DEVICE**

ULTRASCHALLGERÄT

DISPOSITIF ULTRASONOGRAPHIQUE

(84) Designated Contracting States:
**DE FR GB IT NL**

(30) Priority: **25.05.2006  JP 2006145592**

(43) Date of publication of application:
**04.03.2009  Bulletin 2009/10**

(73) Proprietor: **Hitachi Medical Corporation**
**Tokyo 101-0021 (JP)**

(72) Inventors:
• **MATSUMURA, Takeshi**
**Tokyo 101-0021 (JP)**
• **ITO, Ako**
**Ibaraki 317-0077 (JP)**

(74) Representative: **MERH-IP Matias Erny Reichl Hoffmann**
**Patentanwälte PartG mbB**
**Paul-Heyse-Strasse 29**
**80336 München (DE)**

(56) References cited:
**WO-A-2005/120358     WO-A-2005/122907**
**JP-A- 07 055 775     JP-A- 2004 057 653**
**JP-A- 2005 270 341     US-A1- 2006 025 682**
**US-B1- 6 558 324**

• **NAKASHIMA K. ET AL.: 'FLR ni yoru Nyusen Elastography Teiryoka no Rinshoteki Yuyosei' JOURNAL OF MEDICAL ULTRASONICS vol. 33, 15 April 2006, page S134, XP003019607**

**Description**

Technical Field

The present invention relates to an ultrasonic diagnostic apparatus which serves to produce elasticity data such as an elasticity image indicating hardness/softness of the tissue based on a tissue distortion under compression. More specifically, the present invention relates to an ultrasonic diagnostic apparatus which guarantees objectivity and reproducibility of diagnosis of a target portion with respect to benign/malignancy of a target region without a pressure sensor for measuring the stress applied to the tissue.

Background Art

In Patent Document 1, the pressure applied to the skin of the object through the ultrasonic transmission/reception surface of a probe is measured by the pressure sensor, in the diagnosis with an elasticity image of the ultrasonic diagnostic apparatus as described in paragraph [0049]. The pressure is necessary for obtaining the elasticity data and guaranteeing objectivity of the diagnosis with respect to benign/malignancy of the target region using the elasticity image. Also WO 2005120358 A1 discloses an ultrasonic device capable of performing a diagnosis according to an elastic image of a tissue portion. Therein, a pressure is determined by a pressure sensor or a reference deforming body, and an elastic image of the tissue portion is generated based on ultrasonic data and the determined pressure, the generated image being displayed on a display device. Non-Patent Document 1 reports that the correlation between the benign tissue and the malignant tissue with respect to the elasticity modulus is inverted depending on the tissue distortion (hereinafter referred to as compressed state) relevant to the compression in differentiation of the target region based on the elasticity modulus image.

After examination of the related art, inventors of the present invention have noticed the following problems.

That is, the use of the pressure sensor is capable of directly detecting the pressure. However, the diagnosis and the differentiation may further be ensured by allowing measurement of the compressed state of the tissue at the deep portion from the object's skin in contact with the ultrasonic transmission/reception surface.

Patent Document 1: JP2004-261198A

Non-Patent Document 1: Krouskop T, et al.: Elastic Moduli of Breast and Prostate Tissues Under Compression, Ultrasonic Imaging 20: pp. 260-274, 1998

Disclosure of Invention

The present invention provides an ultrasonic diagnostic apparatus including means for evaluating the compressed state of the tissue of the object to be inspected without using the pressure sensor so as to guarantee objectivity and reproducibility of the diagnosis with respect to benign/malignancy of the target portion.

In view of the above objects, an ultrasonic diagnostic apparatus having the features of claim 1 is provided. Preferred embodiments of the invention are described in the dependent claims.

According to an aspect, an ultrasonic diagnostic apparatus includes an elasticity calculation unit for calculating elasticity data of a tissue at plural measurement points based on a pair of frame data of reflection echo signals each measured at a different time, which are obtained in a process where a compressed state of an object is changed, an elasticity image generation unit for generating an elasticity image based on the elasticity data calculated by the elasticity calculation unit so as to be displayed on a display unit, and a compressed state evaluation unit for evaluating the compressed state based on distortion change data of the tissue at the measurement point, wherein the evaluated compressed state is displayed on the display unit correlating with the elasticity image.

In this case, the compressed state evaluation unit is capable of evaluating the compressed state based on a cumulative value ($=\sum \Delta\varepsilon$) of the distortion change of the tissue at the measurement point. The compressed state evaluation unit acquires the distortion change $\Delta\varepsilon$ of the tissue in a set target region from the elasticity calculation unit to obtain a distortion $\varepsilon$ by accumulating values of the distortion change $\Delta\varepsilon$ from a state where the compression state is zero. The compression state evaluation unit acquires the distortion change $\Delta\varepsilon$ of the tissue at the measurement point in a reference region set in a specific tissue to obtain the distortion $\varepsilon$ ($=\sum \Delta\varepsilon$) of the specific tissue based on the distortion change $\Delta\varepsilon$, and a stress $\sigma$ corresponding to the distortion $\varepsilon$ based on a preliminarily measured and stored stress-distortion feature of the specific tissue so as to evaluate the compressed state based on the obtained stress $\sigma$.

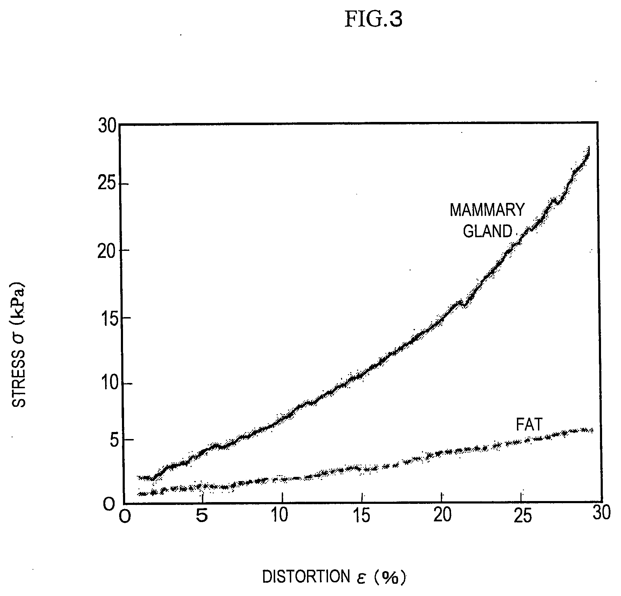

As each stress-distortion feature of the mammary gland and fat tissue in Fig. 3 shows, the distortion $\varepsilon$ ($=\sum \Delta\varepsilon$) as the cumulative value of the distortion change $\Delta\varepsilon$ primarily accords with the stress $\sigma$. Values of the distortion change

$\varepsilon$ as one of data with respect to the small change in the compressed state are accumulated to obtain the distortion $\varepsilon$ so as to evaluate the magnitude of the stress $\sigma$ upon the measurement.

According to another aspect of the present invention, the cumulative value of the distortion change $\varepsilon$ is displayed together with the elasticity image produced based on the small distortion change $\Delta\varepsilon$ so as to evaluate with respect to adequacy of the compressed state. This makes it possible to quantitatively evaluate elasticity of the target portion. The ultrasonic diagnostic apparatus with no pressure sensor may be realized, which guarantees objectivity, reproducibility, and determinacy of the diagnosis with respect to benign/malignancy of a lesion tissue. The compressed state may be evaluated based on not only the distortion $\varepsilon$ but also displacement and stress (absolute amount).

The stress-distortion feature of the tissue may vary depending on the type of the tissue with less individual difference. The stress applied to the tissue is common to the respective portions in the compression direction. The stress-distortion feature of the specific tissue (for example, normal tissue such as fat, muscle, mammary gland) is derived and stored so as to set the reference region of the portion corresponding to the specific tissue upon the measurement. The distortion change $\Delta\varepsilon$ in the reference region is accumulated to obtain the distortion $\varepsilon$ such that the magnitude of the stress $\sigma$ applied to the target portion is estimated based on the stress-distortion feature. The elasticity calculation unit provides the quantitative and objective elasticity data based on the distortion change $\Delta\varepsilon$, the stress $\sigma$, and the stress change $\Delta\sigma$ with respect to the set target region. The elasticity data is a broad term which represents the elasticity, for example, elastic modulus, viscosity coefficient, distortion, stress, distortion ratio, Poisson ratio and the like.

The compressed state evaluation unit is capable of evaluating the compressed state based on the ratio of the distortion changes in two reference regions. That is, the compressed state evaluation unit acquires distortion changes $\Delta\varepsilon1$ and $\Delta\varepsilon2$ corresponding to the reference regions R1 and R2 set at two different tissues from the elasticity calculation unit to obtain the ratio $\Delta\varepsilon2/\Delta\varepsilon1$ ($\Delta\varepsilon1/\Delta\varepsilon2$ is also available, which will apply to the subsequent description). Based on the thus obtained distortion change ratio, the compressed state is evaluated such that the evaluated compressed state is displayed in correlation with the displayed target region.

The stress-distortion feature of the tissue shows different non-linearity which varies with the type of the tissue with less individual difference in the same tissue. The ratio of the distortion changes in the reference regions R1 and R2 set at the two different specific tissues, that is, $\Delta\varepsilon2/\Delta\varepsilon1$ may take the different value depending on the stress $\sigma$ upon the measurement. In other words, the distortion change ratio $\Delta\varepsilon2/\Delta\varepsilon1$ is correlated with the magnitude of the stress applied to the target portion upon measurement, that is, the compressed state of the target portion. This makes it possible to perform the quantitative evaluation with respect to the elasticity in the target portion based on the distortion change ratio $\Delta\varepsilon2/\Delta\varepsilon1$.

The non-linearity feature of the tissue in each of the reference regions R1 and R2 may be approximated to the exponential behavior ($\sigma = \exp(\alpha \times \varepsilon)$). Assuming that the non-linear parameters of the approximate function are set to $\alpha1$ and $\alpha2$, and the stress change $\Delta\sigma$ is applied under the common stress $\sigma$, the relationship of ratio $\Delta\varepsilon2/\Delta\varepsilon1 = \alpha1/\alpha2$ is obtained. If the $\alpha1$ and $\alpha2$ are kept constant, the ratio $\Delta\varepsilon2/\Delta\varepsilon1$ becomes constant, and accordingly the stress $\sigma$ during the measurement cannot be evaluated. The actual measurement of the tissue shows that the value of the $\Delta\varepsilon2/\Delta\varepsilon1$ changes dependent on the stress, indicating incomplete modeling process which fails to accurately express the elasticity response of the actual tissue. Actually, there is the tissue with the compressed state range showing the linear response as the stress-distortion feature. As the compressed state approaches the compression limit of the body tissue, the tissue is not deformed under the large stress change, which hardly causes the distortion change. Accordingly, the non-linearity which can be explained by the exponential behavior as described above is only observed in the local compressed state range. The resultant ratio $\Delta\varepsilon2/\Delta\varepsilon1$ is correlated with the stress $\sigma$ rather than being kept constant, which allows evaluation of the compressed state. In this way, two reference regions showing the correlation with the stress $\sigma$ may be set at the fat and the muscle, the fat and the mammary gland, and the mammary gland and the muscle for examining the breast cancer, for example.

In another aspect of the present invention, the compressed state evaluation unit is capable of obtaining the compressed state corresponding to the distortion change ratio based on a relationship between the preliminarily measured and stored distortion change ratio of two specific tissues $\Delta\varepsilon2/\Delta\varepsilon1$, and the compressed state. Because of the less individual difference in the elasticity with respect to the same kind of tissue, the correlation between the magnitude of the ratio $\Delta\varepsilon2/\Delta\varepsilon1$ and the stress $\sigma$ is stored in the memory as a table corresponding to the specific tissues preliminarily. Then the stress $\sigma$ may be obtained immediately based on the measurement value of the actual distortion change ratio $\Delta\varepsilon2/\Delta\varepsilon1$.

In an example that is useful for understanding the present invention, the broad target region including two reference regions corresponding to the reference region in the foregoing aspect of the present invention and the target region is set. Based on the mean value of the distortion change in the broad target region, the distortion changes of the respective measurement points are normalized. The compressed state evaluation unit according to this example acquires the distortion changes $\Delta\varepsilon1$, $\Delta\varepsilon2$, and $\Delta\varepsilon l$ corresponding to the broad target region which includes the target region and the reference regions R1 and R2 set at the two specific tissues. The mean value $\Delta\varepsilon mean$ of the distortion change in the broad target region is obtained to be output to the elasticity calculation unit. The elasticity calculation unit normalizes

the distortion change $\Delta\varepsilon$ obtained at the measurement point with the mean value to obtain the elasticity data.

The inspector sets the broad target region L-ROI so as to include the target region ROI and reference regions R1, R2 of two different specific tissues. In the case where the reference regions R1 and R2 have a specific positional correlation with the target region ROI in the tissue, for example, the reference regions R1 and R2 are layered while interposing the target region ROI, the reference regions R1 and R2 are so programmed to be automatically set by forming the broad target region L-ROI on the B mode ultrasonogram.

The sum of the distortion changes $\Delta\varepsilon ij$ of all the measurement points Pij in the thus set broad target region is divided by the measurement points Ntot to obtain the mean value $\Delta\varepsilon mean$ of the distortion change in the broad target region. The distortion change $\Delta\varepsilon j$ of the respective measurement points Pij is divided by the mean value $\Delta\varepsilon mean$ of the distortion change in the broad target region to obtain the normalized distortion change $\Delta\varepsilon ij/\Delta\varepsilon mean$ at each of the measurement points P. Based on the normalized distortion change $\Delta\varepsilon ij/\Delta\varepsilon mean$ of the measurement points P, the elasticity image is generated, thus indexing the distortion change $\Delta\varepsilon ij$ of the respective measurement points Pij so as to be displayed.

As the distortion change $\Delta\varepsilon ij/\Delta\varepsilon mean$ at the respective measurement points Pij varies with the applied stress $\sigma$, each elasticity of the respective portions cannot be quantitatively evaluated. Similar to the foregoing aspect of the present invention, the ratio of the distortion changes $\Delta\varepsilon 1$, $\Delta\varepsilon 2$ corresponding to the reference regions R1, R2, that is, $\Delta\varepsilon 2/\Delta\varepsilon 1$ is obtained by the compressed state evaluation unit such that the compressed state is evaluated based on the obtained distortion change ratio, and the evaluated compressed state is displayed on the display unit.

The compressed state evaluation unit is capable of obtaining the compressed state corresponding to the distortion change ratio based on a relationship between the preliminarily measured and stored distortion change ratio of two specific tissues $\Delta\varepsilon 2/\Delta\varepsilon 1$, and the compressed state (for example the stress state).

The compressed state evaluation unit detects a sharp change in the distortion change ratio $\Delta\varepsilon 2/\Delta\varepsilon 1$, and the normalized distortion change $\Delta\varepsilon 1/\Delta\varepsilon mean$ (or $\Delta\varepsilon 2/\Delta\varepsilon mean$) to output a warning on the display unit. The tissue reaches the compression limit where the distortion change hardly occurs while increasing the compression force (stress). The compression force (stress) which reaches the compression limit varies depending on the type of the tissue. When the tissue in one of the reference regions R1 and R2 reaches the compression limit, the distortion change ratio $\Delta\varepsilon 2/\Delta\varepsilon 1$ and the normalized distortion change $\Delta\varepsilon 1/\Delta\varepsilon mean$ or $\Delta\varepsilon 2/\Delta\varepsilon mean$ sharply change. If the tissue in one of the reference regions R1 and R2 reaches the compression limit, the magnitude correlation between the distortion changes $\Delta\varepsilon 1$ and $\Delta\varepsilon 2$ sharply changes. If the sharp changes in the distortion change ratio $\Delta\varepsilon 2/\Delta\varepsilon 1$ and the normalized distortion change $\Delta\varepsilon 1/\Delta\varepsilon mean$ (or $\Delta\varepsilon 2/\Delta\varepsilon mean$) are detected to adjust the compression force (stress), the elasticity image in the predetermined compressed state may be obtained, thus realizing the objective and determinant image diagnosis.

In the present invention, the compressed state may be evaluated based on the small change in the compressed state upon measurement of the elasticity data. This makes it possible to realize the ultrasonic diagnostic apparatus without using a pressure sensor, which guarantees objectivity and reproducibility of the diagnosis with respect to benign/malignancy of the tissue lesion through the compressed state evaluation.

Brief Description of the Drawings

Fig. 1 is a block diagram of an ultrasonic diagnostic apparatus according to an embodiment of the present invention.
Fig. 2 shows an example of an operation performed by a probe to compress the object tissue.
Fig. 3 shows an exemplary graph showing each stress-distortion feature of the mammary gland and the fat tissue.
Fig. 4 is a flowchart of the process performed in a first embodiment.
Fig. 5 is a view showing an example of a display screen according to the first embodiment.
Fig. 6 is a view showing an example of a display screen according to a second embodiment.
Fig. 7 shows an example of the stress-distortion feature of the fat portion in the second embodiment.
Fig. 8 is a flowchart of the process performed in a third embodiment.
Fig. 9 is a view showing an example of a display screen according to the third embodiment.
Fig. 10 shows an example of each stress-distortion feature of the fat, muscle, and the target portion.
Fig. 11 shows the stress-distortion feature in the graph where the x-axis and y-axis of the graph shown in Fig. 10 are inverted.
Fig. 12 is a view of the stress-distortion feature which shows the compression limit.
Fig. 13 is a view of the stress-distortion change ratio which shows the compression limit of the tissue.
Fig. 14 is a view showing an example of an elasticity modulus image according to a fourth embodiment.
Fig. 15 is a flowchart of the process performed in a fifth embodiment.
Fig. 16 is a view showing an example of a normalized distortion image according to the fifth embodiment.
Fig. 17 shows a behavior of the normalized distortion change at the compression limit.

Fig. 18 shows an example of a color map showing the hue which represents the normalized distortion change under the stress.

Fig. 19 shows another example of the color map showing the hue which represents the normalized distortion change under the stress.

Fig. 20 shows a distribution of the normalized distortion change in a depth direction.

Fig. 21 shows an example where the gradient of the distortion change distribution in the depth direction changes from the negative direction to the positive direction as the compression is intensified.

Fig. 22 is a view for explaining an eighth embodiment where the stress is evaluated for each line region.

Fig. 23 is a view for explaining a method according to an eleventh embodiment for setting the reference region in the broad target region.

Fig. 24 is a view for explaining a method according to a twelfth embodiment for setting the reference region using a template.

Fig. 25 is a view for explaining a method according to a thirteenth embodiment for automatically setting the reference region.

Fig. 26A shows an example where the reference region deviates from the tissue which is deformed under the compression.

Fig. 26B shows an example where the reference region is moved and changed to follow deformation of the tissue under the compression.

Fig. 26C shows details of the example where the reference region is moved and changed to follow the deformation of the tissue under the compression.

Fig. 26D shows the reference region before/after the movement and change shown in Fig. 26C.

Fig. 27 shows an elasticity image displayed by the ultrasonic diagnostic apparatus of the present invention according to a seventeenth embodiment in a preferred form.

Fig. 28 shows a nineteenth embodiment where the stress-distortion correlation in the target region ROI of the target portion is plotted real-time to form the graph.

Fig. 29 shows an example for displaying the zone corresponding to the type of the tissue on the stress-distortion graph in the nineteenth embodiment.

Fig. 30 shows an experimental results which allow differentiation with respect to benign/malignancy with high accuracy when the elasticity is indexed using the value of the distortion change ratio ($\Delta\varepsilon2/\Delta\varepsilon1$) as the stress index of $\sigma$index.

Best Mode for Carrying Out the Invention

Embodiments of an ultrasonic diagnostic apparatus according to the present invention will be described.

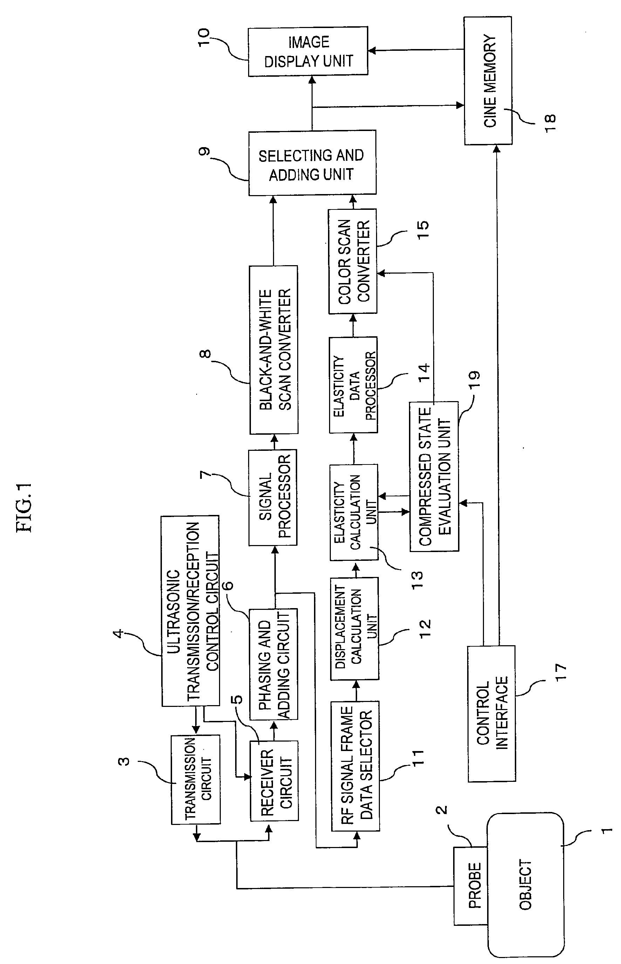

Fig. 1 is a block diagram of the ultrasonic diagnostic apparatus according to an embodiment of the present invention. Referring to the drawing, an ultrasonic probe 2 used in contact with the skin of a object 1 includes an ultrasonic transmission/reception surface having a plurality of transducers arranged for transmission/reception of the ultrasonic wave to/from the object 1. The probe 2 is driven by the ultrasonic pulse supplied from a transmission circuit 3. An ultrasonic transmission/reception control circuit 4 controls a transmission timing of an ultrasonic pulse for driving the plurality of transducers of the probe 2 so as to form the ultrasonic beam to the focus set inside the object 1. The ultrasonic transmission/reception control circuit 4 is structured to electronically scan the ultrasonic beam in the direction where the transducers are arranged in the probe 2.

Meanwhile, the probe 2 receives a reflection echo signal generated in the object 1 so as to be output to a receiver circuit 5. The receiver circuit 5 acquires the reflection echo signal and performs the reception process such as amplification in accordance with the timing signal input from the ultrasonic transmission/reception control circuit 4. The reflection echo signal performed to the reception process by the receiver circuit 5 is amplified by a phasing and adding circuit 6 for aligning a phase and adding the reflection echo signals received by the plurality of transducers. The RF signal of the reflection echo signals aligned the phase and added by the phasing and adding circuit 6 is input to a signal processor 7 for performing the signal processing such as gain correction, log compression, phase detection, edge enhancement, and filtering. The RF signal generated by the phasing and adding circuit 6 may be composite demodulated I, Q signals.

The RF signal processed in the signal processor 7 is sent to a black-and-white scan converter 8 where the signal is converted into a digital signal as well as converted into a two-dimensional ultrasonogram data corresponding to the scan plane of the ultrasonic beam. The signal processor 7 and the black-and-white scan converter 8 constitute image re-structuring means of a ultrasonogram (B mode image). The ultrasonogram data output from the black-and-white scan converter 8 are supplied to an image display unit 10 via a selecting and adding unit 9 such that the B mode image is displayed.

Meanwhile, the RF signal output from the phasing and adding circuit 6 is sent to an RF signal frame data selector 11. The RF signal frame data selector 11 acquires frame data of a plurality of frames such that the group of RF signals corresponding to the scan plane (ultrasonogram plane) of the ultrasonic beam is stored in the memory. A displacement calculation unit 12 acquires plural pairs of frame data at different acquiring time stored in the RF signal frame data selector 11 sequentially. The displacement vector at the plurality of measurement points on the tomogrpahic plane is obtained based on the acquired pair of frame data so as to be output to an elasticity calculation unit 13 as the displacement frame data.

The elasticity calculation unit 13 according to the embodiment serves to generate distortion frame data by obtaining the distortion change of the tissue at the measurement points based on the displacement frame data, and to calculate the other elasticity data. An elasticity data processor 14 is structured to perform various image processing operations with respect to the frame data of the elasticity data output from the elasticity calculation unit 13, for example, smoothing process on the coordinate plane, contrast optimizing process, and smoothing process along the time axis between frames. A color scan converter 15 acquires the frame data of the elasticity data output from the elasticity data processor 14 to generate a color elasticity image by allocating a tone code to each pixel of the frame data in accordance with the color map of the set elasticity data.

The color elasticity image generated by the color scan converter 15 is displayed on the image display unit 10 via the selecting and adding unit 9. The selecting and adding unit 9 serves to receive ultrasonogram output from the black-and-white scan converter 8 and the color elasticity image output from the color scan converter 15 for selecting the image therebetween to be displayed, to make one of the images translucent so as to be objected to addition and synthesis for performing superimposing display on the image display unit 10, and to display both images. A cine memory 18 stores image data output from the selecting and adding unit 9, and calls the previous image data so as to be displayed on the image display unit 10 upon the command of a control interface 17. The selected image data may further be transferred to a recording medium such as an MO.

A compressed state evaluation unit 19 as an essential portion of the present invention serves to evaluate the state of the compressed object based on the data with respect to the small change in the distortion amount upon the measurement. Detailed explanations with respect to the structure of the compressed state evaluation unit 19 and the correlation between the elasticity calculation unit 13 and the color scan converter 15 will be described in the subsequent embodiment.

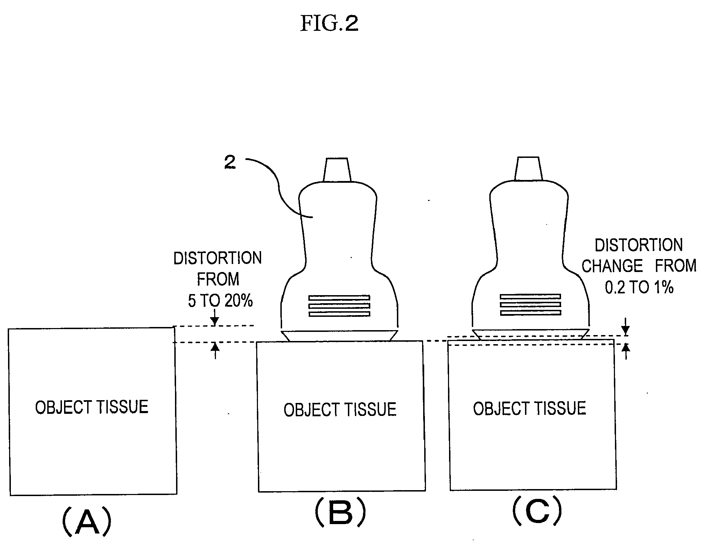

The basic operation of the above-structured embodiment will be described. An example of a compression applied to the object 1 by the probe 2 will be described referring to Figs. 2(A) to (C). Basically, compression is applied to the object 1 by the probe 2 in contact therewith, and the absolute stress $\sigma$ applied to the body tissue is changed by a small stress change of $\Delta\sigma$ so as to cause the small distortion change $\Delta\varepsilon$ by 0.2 to 1 % in the state where the absolute distortion $\varepsilon$ by 5 to 20% occurs. The ultrasonic beam is scanned in the object 1 while repeating the small stress change $\Delta\sigma$ for sequentially receiving the reflection echo signals from the scan plane. Based on the RF signal output from the phasing and adding circuit 6, the ultrasonogram (B mode) is re-constructed by the signal processor 7 and the black-and-white scan converter 8 so as to be displayed on the image display unit 10 via the selecting and adding unit 9.

Meanwhile, the RF signal frame data selector 11 acquires the RF signal in the state where the compression applied to the object 1 varies to repeatedly obtain the frame data in synchronization with the built-in frame rate so as to be stored in the frame memory in time series. Plural pairs of frame data each as a unit formed of a pair of reflection echo signals acquired at a different timing are sequentially selected and output to the displacement calculation unit 12. The displacement calculation unit 12 processes the selected pair of frame data to one-dimensional correlation or two-dimensional correlation, measure, the displacement at the respective measurement points on the scan plane, generates displacement frame data. The known block matching process may be employed as the process for detecting the displacement vector. That is, the image is divided into blocks each formed of N × N pixels, and the block which most approximates the target block in the current frame is searched, based on which the displacement of the measurement point is obtained. The self correlation in the same region of the pair of RF signal frame data is calculated to obtain the displacement.

The displacement frame data obtained by the displacement calculation unit 12 are input to the elasticity calculation unit 13 where the preliminarily set elasticity data such as each distortion change at the respective measurement points is calculated, and the required elasticity frame data are output to the elasticity data processor 14. The distortion change may be calculated by performing space deviation of the displacement through the known process.

The elasticity calculation unit 13 acquires compressed evaluation data output from the compressed state evaluation unit 19 to obtain the elasticity data which is quantified as needed. The obtained elasticity data is input to the color scan converter 15 via the elasticity data processor 14 to generate and display the elasticity image on the image display unit 10.

Details of structures of the compressed state evaluation unit 19 as the essential portion of the present invention, and the elasticity calculation unit 13, the color scan converter 15, and the control interface 17 as related components will be described as well as operations thereof based on the specific embodiments.

First Embodiment

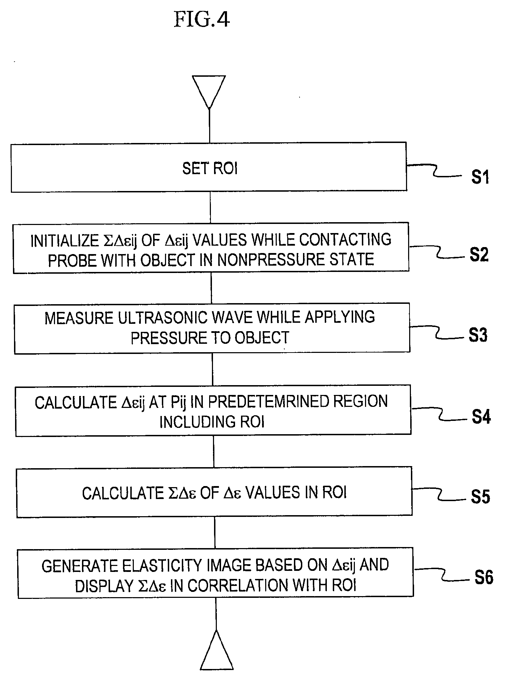

In an embodiment, the compressed state is evaluated using a cumulative value $\sum\Delta\varepsilon$ (=distortion $\varepsilon$) derived from summing the distortion changes $\Delta\varepsilon$ in the target region from the state where no compression is applied. Fig. 4 is a flowchart of the process performed in the embodiment.

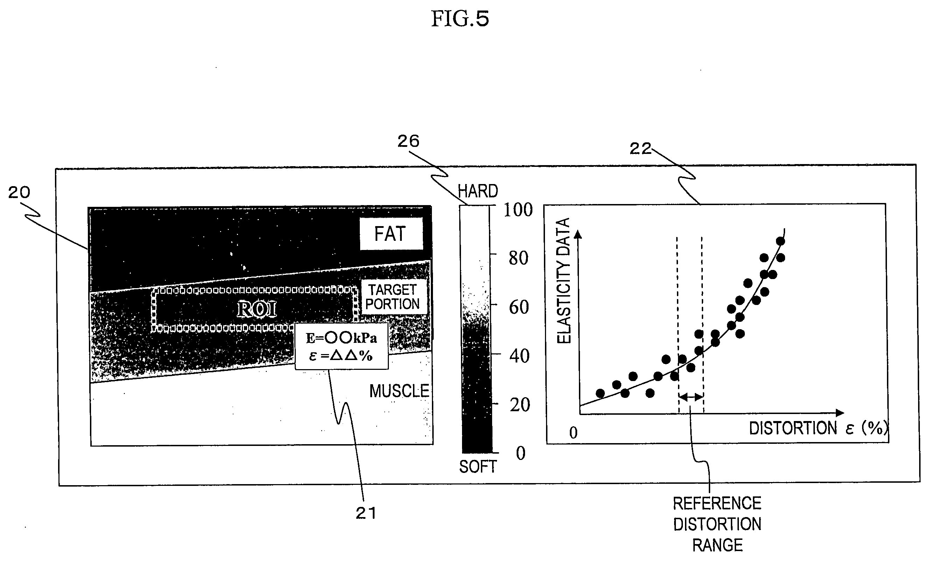

In step S1, the target region ROI which contains the target tumor and the like using the B mode image or the elasticity image displayed on the image display unit 10 as shown in Fig. 5. Fig. 5 shows a distortion image 20 as the elasticity image. The normalized distortion change image to be described later may be displayed as the distortion image 20.

In step S2, the probe is brought into contact with the skin of the object, and the cumulative value $\sum\Delta\varepsilon ij$ of the distortion change stored in the compressed state evaluation unit 19 from the control interface 17 is initialized (reset) to zero in the state where no compression is applied. The uncompressed state may be detected when starting input of the reflection echo signal. Thereafter, in step S3, the initial compression is applied to the object 1 (Fig. 2(B)), and the ultrasonic wave is measured (Fig. 2(C)) under the compression while being changed by a small amount. In step S4, the small distortion change $\Delta\varepsilon ij$ at each measurement point in the predetermined region which contains the target region ROI is obtained by the elasticity calculation unit 13. In step S5, simultaneously with execution of step S4, the compressed state evaluation unit 19 obtains the cumulative value $\sum\Delta\varepsilon$(=distortion $\varepsilon$) of the small distortion change $\Delta\varepsilon$ in the target region ROI from the uncompressed state. The color scan converter 15 generates a color distortion image 20 real-time based on the distortion change $\Delta\varepsilon ij$ output from the elasticity calculation unit 13. Then the color distortion image 20 is displayed on the image display unit 10 as the left drawing in Fig. 5 shows. The distortion $\varepsilon$ output from the compressed state evaluation unit 19 is displayed by a numerical value 21 corresponding to the ROI (S6). The level of the elasticity showing rigidity and flexibility of each portion of the color distortion image 20 is shown in correlation with the tone of a color bar 26. The measured distortion changes $\Delta\varepsilon$ and the resultant distortions $\varepsilon$ (%) are plotted to form the distortion change $\Delta\varepsilon$ - distortion $\varepsilon$ chart 22 as shown in Fig. 5, which are arranged and displayed with the elasticity image on the image display unit 10. It is preferable to plot those data into the normalized distortion change $\Delta\varepsilon norm$ - distortion s chart to be described later with higher objectivity and reproducibility rather than the distortion change $\Delta\varepsilon$ - distortion $\varepsilon$ chart.

The cumulative value $\sum\Delta\varepsilon$ of the distortion change $\Delta\varepsilon$ in the embodiment represents the distortion $\varepsilon$ accumulated from the uncompressed state, which is correlated with the stress $\sigma$ based on the stress - distortion feature of the tissue in the ROI. This makes it possible to evaluate the color displayed elasticity of the displayed elasticity image in reference to the magnitude of the distortion $\varepsilon$. The appropriate reference distortion range is displayed on the distortion change $\Delta\varepsilon$ - distortion $\varepsilon$ chart so as to allow the inspector to perform the quantitative and reproducible diagnosis with respect to the elasticity of the tissue in the ROI under the appropriate stress.

The distortion change $\Delta\varepsilon$ is the value dependent on the externally applied compression (displacement amount of the probe 2). The elasticity image generated based on the distortion change only indicates the relative relationship between elasticity values among the tissues but fails to provide the elasticity data which shows the elasticity unique to the tissue quantitatively. As disclosed in Non-Patent Document 1, the elasticity of the tissue exhibits the non-linearity as shown by the stress - distortion charts of the mammary gland and the fat tissue in Fig. 3. The tissue becomes harder as the absolute distortion $\varepsilon$ (hereinafter simply referred to as the distortion) becomes large. The elastic modulus is represented by the gradient of the stress-distortion chart to take the quantitative value unique to the tissue. However, the elastic modulus varies with the compressed state represented by the absolute stress (hereinafter referred to as the stress) $\sigma$ or the distortion $\varepsilon$. As the elastic modulus unique to the tissue varies with the compressed state (stress $\sigma$, distortion s), the elastic image formed based on the elasticity data will be relatively changed depending on the compressed state as well. As the cumulative value $\sum\Delta\varepsilon$(=$\varepsilon$) is correlated with the stress $\sigma$ in the embodiment, the elasticity unique to the tissue may be quantitatively evaluated based on the magnitude of the stress.

Besides the distortion and the elastic modulus, the elasticity data according to the embodiment may be the viscosity coefficient, distortion ratio, and Poisson ratio. A plurality of target regions ROI may be set such that the elasticity images, the elasticity data - distortion $\varepsilon$ chart, and the elasticity data - stress $\sigma$ chart may be formed corresponding to the respective regions ROI.

Second Embodiment

The stress-distortion feature of the tissue varies dependent on its type with less individual difference. The stress-distortion feature of the specific tissue (normal tissue, for example, fat and muscle) other than the target portion is preliminarily stored in the memory, and the reference region R1 corresponding to the specific tissue upon measurement is set. The distortion cumulative value $\sum\Delta\varepsilon 1$ in the reference region R1 allows estimation of the magnitude of the stress $\sigma$ which acts on both the target region and the reference region based on the stress-distortion feature of the specific tissue. Estimated magnitude of the stress $\sigma$ provides the elasticity data of the target portion such as the elastic modulus E determinately. The second embodiment will be described in detail hereinafter.

Generally, the body tissue exhibits the stress-distortion feature of the mammary gland portion as shown in Fig. 3, indicating the non-linear elasticity feature relative to the distortion. The response may be analyzed using the exponent function generally expressed as the following equation (1).

$$\sigma = \exp(\alpha \times \varepsilon) \quad (1)$$

The analytical result of approximating (fitting) the actual measurement value of the stress-distortion of the tissue through the equation (1) is reflected on the term $\alpha$ therein. As the value of $\alpha$ becomes large, the non-linearity becomes more noticeable. Accordingly, the term $\alpha$ is referred to as a non-linear parameter (see Non-Patent Document 3).

When differentiating the equation (1) with respect to the term $\varepsilon$, the following equation (2) is established.

$$(d\sigma/d\varepsilon) = \alpha \times \exp(\alpha \times \varepsilon) = \alpha \times \sigma \quad (2)$$

The small stress change $\Delta\sigma$ and the resultant small distortion change $\Delta\varepsilon$ are correlated as expressed by the following equation (3).

$$\Delta\sigma = \alpha \times \sigma \times \Delta\varepsilon \quad (3)$$

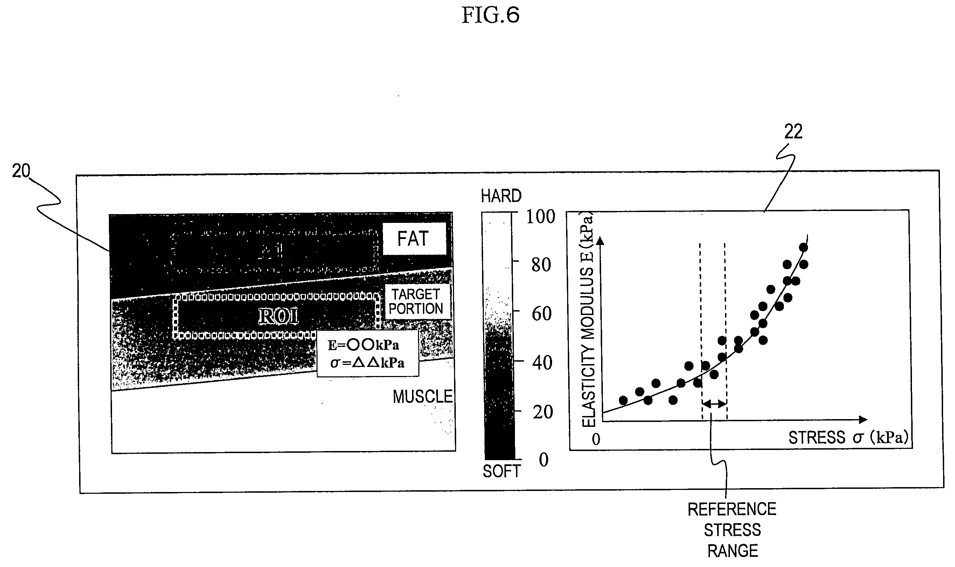

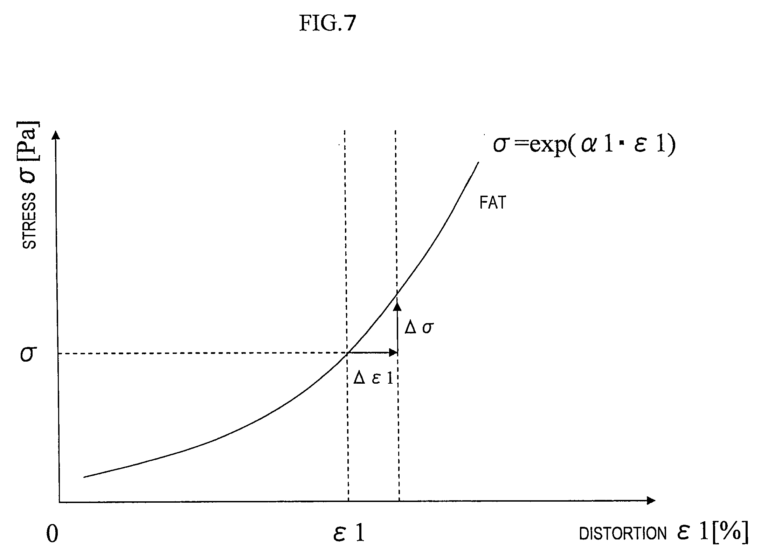

The process for obtaining the value $\Delta\sigma$ of the equation (3) from the specific tissue other than the target portion will be described referring to Fig. 6. Assuming that the reference region R1 shown in the drawing is set in the fat portion, the term $\alpha$ of the equation (3) is derived from the stress-distortion feature of fat so as to be referred to as $\alpha1$. Referring to Fig. 7, the distortion change measured in the reference region R1 and the distortion as the cumulative value will be referred to as $\Delta\varepsilon1$ and $\varepsilon1=\sum\Delta\varepsilon1$, respectively. The stress $\sigma$ which acts on both the target region and the reference region is obtained as $\sigma = \exp(\alpha1 \times \varepsilon1)$ through the equation (1). The common stress change at this time may be obtained as $\Delta\sigma = \alpha1 \times \sigma \times \Delta\varepsilon1$ through the equation (3).

Generally, the stress is divided by the distortion to employ Young's modulus as the elastic modulus E. The following equation (4) may be used to obtain Young's modulus in the target region comprehensively.

$$E = \Delta\sigma/\Delta\varepsilon I \quad (4)$$

where $\Delta\varepsilon I$ denotes the distortion change measured in the target region.

In the aforementioned case, the right chart 22 in Fig. 6 may be expressed as the elastic modulus E - stress $\sigma$ chart which represents the compressed state correlated with the elastic modulus. When forming the elastic modulus image in accordance with the aforementioned process, the equation (4) is used for each of the measurement points Pij to create the image of the elastic modulus calculated as $Eij=\Delta\sigma/\Delta\varepsilon ij$.

In the example shown in Figs. 6 and 7, the stress $\sigma$ is obtained based on the non-linear parameter $\alpha$. However, the stress $\sigma$ may be directly derived from $\sum\Delta\varepsilon1$ by forming the feature data shown in Fig. 7 into the table so as to be stored in the memory. As a result, the embodiment is applicable to the case where the non-linear parameter $\alpha$ is not set as the constant.

In the present embodiment and the first embodiment, the cumulative value $\sum\Delta\varepsilon ij$ of the distortion change stored in the compressed state evaluation unit 19 from the control interface 17 is required to be initialized (reset) to zero in the state where the probe is brought into contact with the skin of the object under the uncompressed state. The aforementioned reset operation is arbitrarily performed by the inspector. When the reset operation is delayed, the cumulative value $\sum\Delta\varepsilon ij$ may contain the error. The determinacy and producibility of the present embodiment are inferior to those of the process for evaluating the compressed state using the small distortion change ratio $\Delta\varepsilon2/\Delta\varepsilon1$ only under the arbitrary compressed state to be described in the following third and fourth embodiments.

Third Embodiment

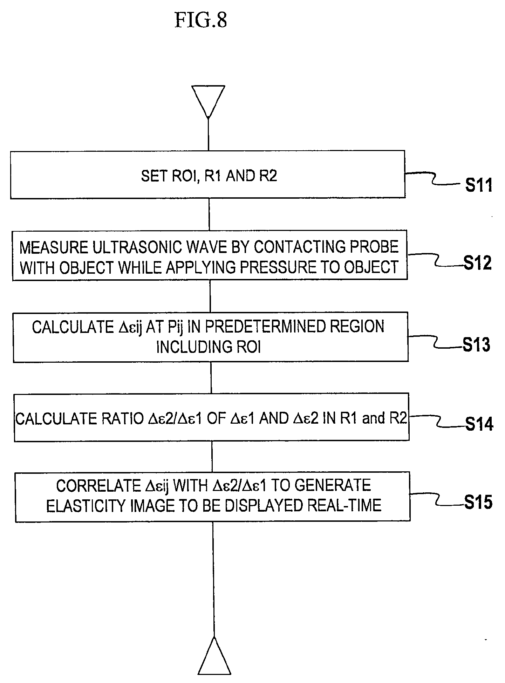

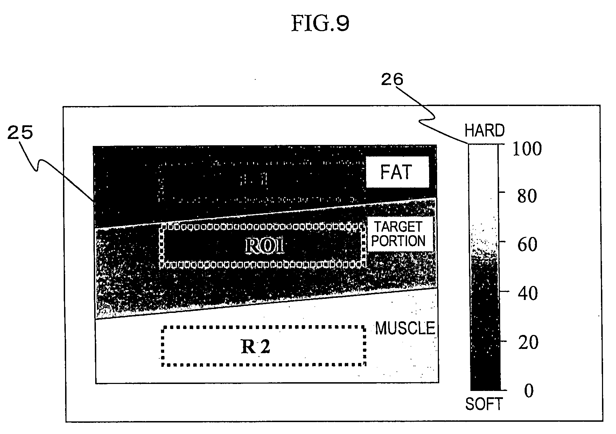

In the embodiment, the compressed state is evaluated using the distortion change ratio $\Delta\varepsilon2/\Delta\varepsilon1$ of two reference regions R1 and R2 set at the specific tissues other than the target region. Fig. 8 is a flowchart of the process performed in the embodiment. Fig. 9 shows an example of a display of a distortion image 25 according to the embodiment

Referring to Fig. 8, in step S11, the target region ROI which contains the target lesion site is set using an image 25 derived from picking up the target site, for example, the B mode image or the distortion image as shown in Fig. 9. The reference regions R1 and R2 are set in predetermined different specific tissues. In Fig. 9, the upper portion as fat and the lower portion as muscle are layered while interposing the target portion such as the mammary gland for diagnosing the breast cancer. The target region ROI is set in the target portion, the reference region R1 is set in the fat portion as the normal tissue, and the reference region R2 is set in the muscle portion as the normal tissue which is different from the fat portion. Thereafter, in step S12, the probe is brought into contact with the skin of the object to apply the initial compression to the object 1 (Fig 2(B)). Then the ultrasonic wave is measured by applying very low pressure while being changed (Fig. 2(C)).

Upon measurement of the ultrasonic wave, the small distortion change $\Delta\varepsilon ij$ at each of the measurement points in the whole measurement region which contains the target region ROI, and the reference regions R1, R2 is obtained by the elasticity calculation unit 13 (S13). The compressed state evaluation unit 19 obtains the small distortion change ratio $\Delta\varepsilon 2/\Delta\varepsilon 1$ in the reference regions R1 and R2 (S14) simultaneously with execution of step S13. The color scan converter 15 produces the color distortion image real-time based on the distortion change $\Delta\varepsilon ij$ output from the elasticity calculation unit 13 to display the elastic image on the image display unit 10 (step S15). Instead of the distortion image, it is preferable to form the normalized distortion image and the elastic modulus image so as to be displayed.

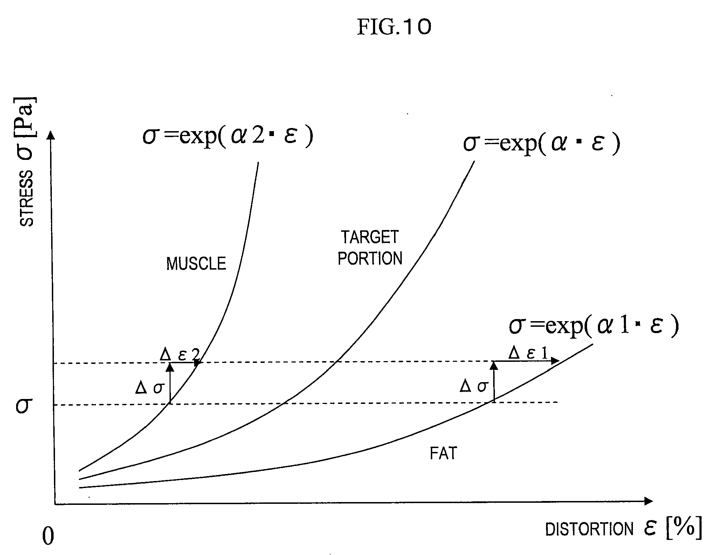

For example, as shown in Fig. 9, the reference region R1 is set in the relatively soft fat portion, the reference region R2 is set in the relatively hard muscle portion of the great pectoral muscle, and the target region ROI is set in the target portion. Each tissue of the fat, muscle, and the target portion exhibits the stress-distortion feature as shown in Fig. 10, respectively. Assuming that each stress-distortion feature in the reference regions R1 and R2 is modeled to be expressed as the exponent functions expressed by the following equations (5) and (6) as described in the second embodiment. In the aforementioned equations, the terms $\alpha 1$ and $\alpha 2$ are non-linear parameters (constants) each indicating the non-linearity of the tissue in the reference regions R1 and R2, respectively.

$$\sigma = \exp(\alpha 1 \times \varepsilon) \quad (5)$$

$$\sigma = \exp(\alpha 2 \times \varepsilon) \quad (6)$$

Likewise the second embodiment, assuming that the distortion values in the reference regions R1 and R2 are set to $\varepsilon 1$ and $\varepsilon 2$, the common stress change under the stress $\sigma$ which acts on both the reference region and the target region may be obtained through the following equation (7).

$$\Delta\sigma = \alpha 1 \times \sigma \times \Delta\varepsilon 1, \quad \Delta\sigma = \alpha 2 \times \sigma \times \Delta\varepsilon 2 \quad (7)$$

The distortion change ratio $\Delta\varepsilon 2/\Delta\varepsilon 1$ may be obtained through the following equation (8).

$$\Delta\varepsilon 2/\Delta\varepsilon 1 = \alpha 1/\alpha 2 \quad (8)$$

As the equation (8) shows no dependency of $\Delta\varepsilon 2/\Delta\varepsilon 1$ on the stress $\sigma$, the compressed state cannot be evaluated based on the value of $\Delta\varepsilon 2/\Delta\varepsilon 1$. However, actual measurement of the tissue shows that the value of the $\Delta\varepsilon 2/\Delta\varepsilon 1$ varies dependent on the stress $\sigma$. This shows that incomplete modeling process fails to accurately express the elastic response of the tissue.

The correlation of the value of $\Delta\varepsilon 2/\Delta\varepsilon 1$ with the stress $\sigma$ of the actual tissue will be described with respect to two behaviors among those of the elastic response of the tissue.

(behavior 1) The tissue has the range of the compressed state indicating the linear response as the stress-distortion feature.
(behavior 2) Because of the compression limit in the body tissue, as the compression gets close to the limit, the tissue is hardly deformed under the large stress change, and the distortion change is unlikely to occur.

The "behavior 1" will be described in the embodiment, and the "behavior 2" will be described in the subsequent embodiment.

(behavior 1)

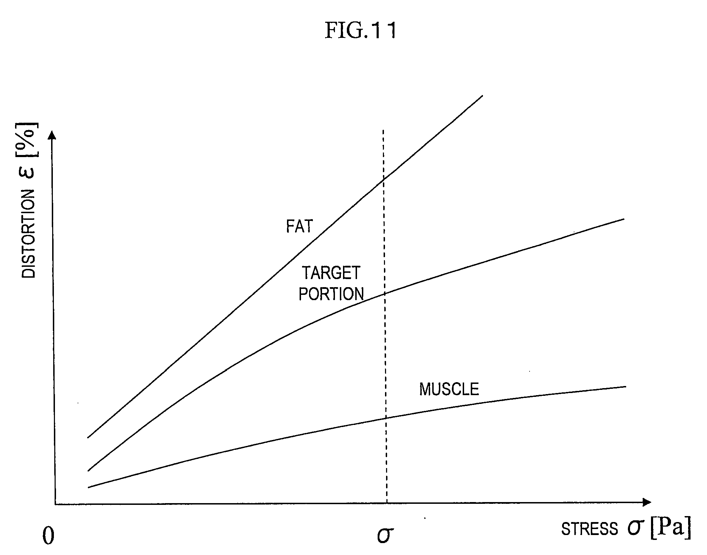

The fat portion in the reference region R1 has relatively lower non-linearity than any other tissue. Referring to Fig. 3 and Non-Patent Document 1, the stress-distortion feature of the reference region R1 may be approximated to the linear feature as shown in Fig. 11 in the normal measurement range up to the distortion of 30%. The gradient of the distortion $\varepsilon$ relative to the stress $\sigma$ to the fat portion is expressed as the constant a to establish the following equation (9).

$$\sigma = a \times \varepsilon \quad (9)$$

When the aforementioned relationship is construed as the function of the stress, it may be modified into the following equations (10) and (11).

$$\varepsilon = (1/\alpha 1) \times \ln(\sigma), \quad (\Delta\varepsilon/\Delta\sigma) = (1/\alpha 1) \times (1/\sigma) \quad (10)$$

$$\varepsilon = (1/\alpha 2) \times \ln(\sigma), \quad (\Delta\varepsilon/\Delta\sigma) = (1/\alpha 2) \times (1/\sigma) \quad (11)$$

Based on the equations (10) and (11), the distortion changes $\Delta\varepsilon 1$ and $\Delta\varepsilon 2$ in the reference regions R1 and R2 may be expressed as the following equations (12) and (13).

$$\Delta\varepsilon 1 = (1/a) \times \Delta\sigma \quad (12)$$

$$\Delta\varepsilon 2 = (1/\alpha 2) \times (1/\sigma) \times \Delta\sigma \quad (13)$$

The stress change $\Delta\sigma$ common to the target region and the reference region is erased from each of those equations (12) and (13), and the relationship between the distortion change ratio $\Delta\varepsilon 2/\Delta\varepsilon 1$ and the stress $\sigma$ may be expressed by the following equation (14).

$$\Delta\varepsilon 2/\Delta\varepsilon 1 = (a/\alpha 2) \times (1/\sigma)$$

$$\sigma = (a/\alpha 2) \times (\Delta\varepsilon 1/\Delta\varepsilon 2) \quad (14)$$

As the equation (14) clearly shows, the distortion change ratio $\Delta\varepsilon 2/\Delta\varepsilon 1$ is primarily correlated with the stress $\sigma$ as one of the evaluation indexes under the compression. That is, the compressed state upon the measurement may be evaluated using the measurement value of the distortion change ratio $\Delta\varepsilon 2/\Delta\varepsilon 1$ upon the arbitrary measurement.

Likewise the second embodiment, because of less individual difference in the elastic response of the specific tissue, constants a and $\alpha 2$ of the equation (14) may be set to the known values as actual measurement data shown in Fig. 3. The use of the values provides the value of the stress $\sigma$ representing the compressed state. Likewise the process in the second embodiment, the common stress change $\Delta\sigma$ may be obtained, thus allowing the elastic modulus E of the target region and the elastic modulus Eij for each measurement point to be obtained.

In step S15 of Fig. 8, the value of the distortion change $\Delta\varepsilon ij$ at the measurement point which includes the target region ROI is correlated with the distortion change ratio $\Delta\varepsilon 2/\Delta\varepsilon 1$ upon the measurement to realize the color gradation for generating the color distortion image 25 as shown in Fig. 9. The elasticity level of the respective portions of the color distortion image 25 indicating hardness/softness thereof may be shown corresponding to the respective tones of the color bar 26.

In the present embodiment, the inspector is able to compare the color distortion image evaluated based on the compressed state with the color bar 21, objectively diagnose the elasticity of the target portion.

In the explanation, the distortion image 25 is explained as the elasticity image. However, the elastic modulus may be employed without being limited to the distortion image. The explanation is made on the assumption that the target region ROI is set for obtaining the numeric data with respect to the elasticity data of the target portion. However, the target region ROI does not have to be set so long as the elasticity image which contains the elastic modulus image is intended to be displayed.

In the embodiment, the value of the representative points among the measurement points in the reference regions R1 and R2 may be used as the distortion change values $\Delta\varepsilon1$ and $\Delta\varepsilon2$. However, mean values $\Delta\varepsilon1$mean and $\Delta\varepsilon2$mean of the respective measurement points in the reference regions R1 and R2 may be used without being limited to the aforementioned values.

Fourth Embodiment

The fourth embodiment describes the (behavior 2) of the elastic response of the tissue in the case where the value of $\Delta\varepsilon2/\Delta\varepsilon1$ is correlated with the stress $\sigma$ in the actual tissue as described in the third embodiment.

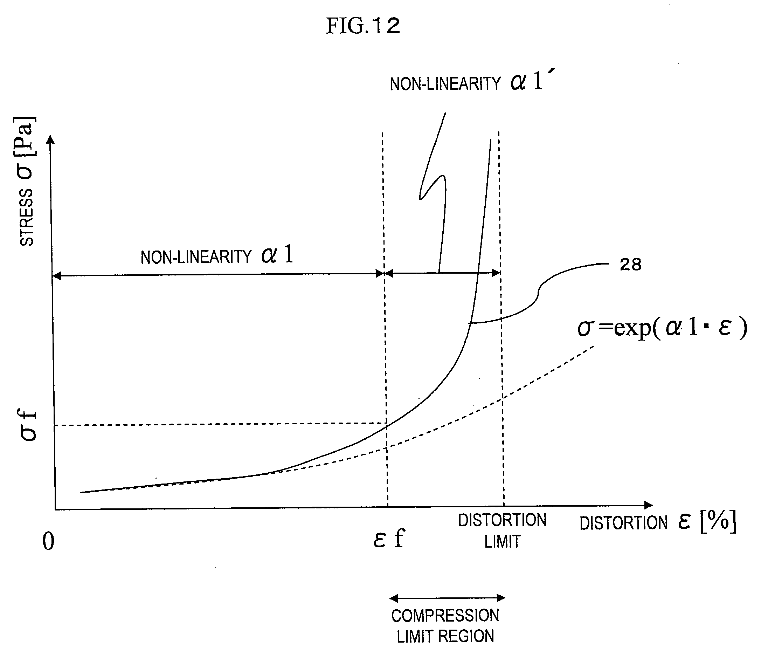

The stress-distortion feature of the actual tissue is not formed as the simple model as expressed by the equations (5) and (6), and the non-linear parameters $\alpha1$ and $\alpha2$ are correlated with the magnitude of the stress and the distortion. As the stress $\sigma$ applied to the tissue is increased, the distortion state reaches a certain point where the tissue is unlikely to be deformed irrespective of the large stress change $\Delta\sigma$, and the distortion change $\Delta\varepsilon$ hardly occurs as shown by a curve 28 of Fig. 12. The curve 28 in Fig. 12 shows the case of fat where the non-linear parameter $\alpha1$ has changed to $\alpha1'$ ($\alpha1'>>(\alpha1)$) at a timing before/after the aforementioned phenomenon occurs. The region where the aforementioned phenomenon occurs is referred to as a compression limit region having the boundary stress of $\sigma f$, and the boundary distortion of $\varepsilon f$. The boundary stress $\sigma f$ varies dependent on the type of the tissue. When the tissue in one of the reference regions R1 and R2 reaches the compression limit, the distortion change ratio $\Delta\varepsilon2/\Delta\varepsilon1$ sharply changes as shown by a curve 29 of Fig. 11. That is, the ratio $\Delta\varepsilon2/\Delta\varepsilon1$ changes in correlation with the stress $\sigma$ indicating the compressed state.

If the inspector is warned of detection of the sharp change in the distortion change ratio $\Delta\varepsilon2/\Delta\varepsilon1$, the measurement may be performed in the compressed state range where the tissue in one of the reference regions R1 and R2 does not reach the compression limit by adjusting the compression force. The inspector is allowed to measure the distortion change $\Delta\varepsilon I$ in the target region ROI in the range of the predetermined compressed state, and to diagnose the target portion using the color distortion image under the predetermined compressed state.

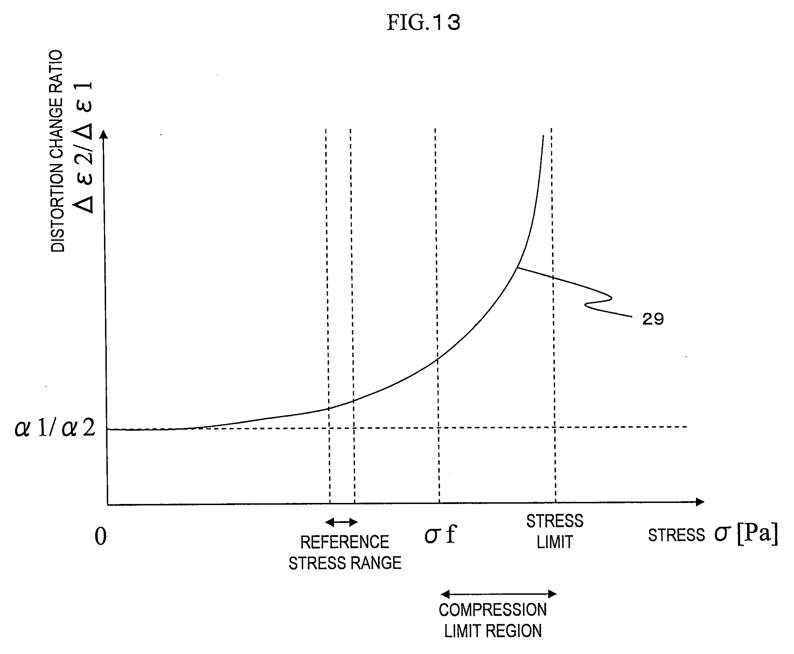

In the embodiment, the stress-distortion feature in the range which contains the compression limit region shown in Fig. 12 and a feature curve 29 of the $\sigma$-$\Delta\varepsilon2/\Delta\varepsilon1$ shown in Fig. 13 are preliminarily measured, based on which the stress $\sigma$ corresponding to the distortion change ratio $\Delta\varepsilon2/\Delta\varepsilon1$ upon the measurement is directly estimated. Referring to Fig. 13, in the stress range where the stress does not reach the boundary stress $\sigma f$, the distortion change ratio $\Delta\varepsilon2/\Delta\varepsilon1$ varies with the stress $\sigma$. This makes it possible to primarily estimate the stress $\sigma$ upon the measurement from the distortion change ratio $\Delta\varepsilon2/\Delta\varepsilon1$ based on the feature curve 29. The values of $\alpha1$ (or a) and $\alpha2$ are preliminarily measured and stored in the memory as the feature data of the feature curve 29 such that the compressed state evaluation unit 19 estimates the stress $\sigma$ through the equation (14) using the value of $\Delta\varepsilon2/\Delta\varepsilon1$.

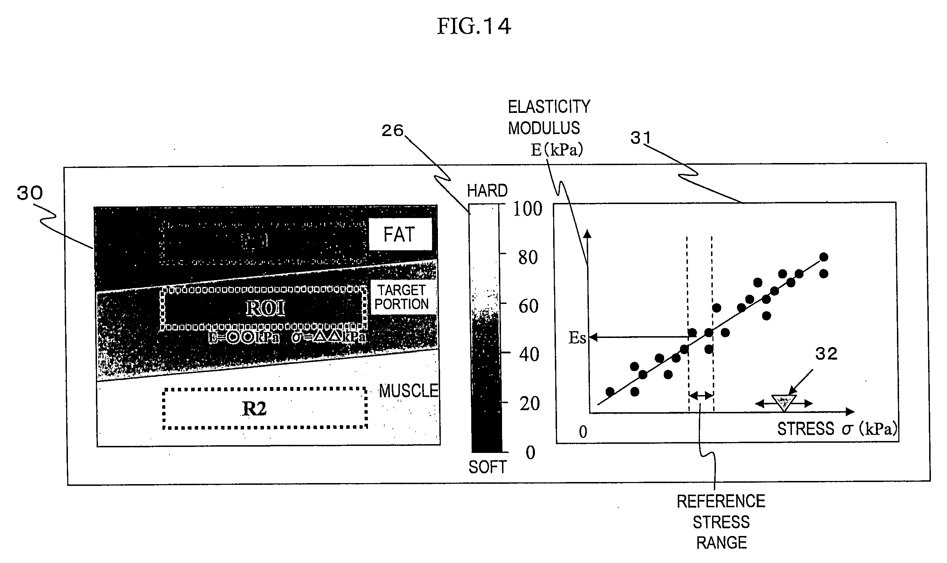

The elasticity data of the target portion such as the elastic modulus and the viscosity coefficient may be obtained from the distortion change $\Delta\varepsilon I$ in the target region ROI using the estimated value of the stress $\sigma$ upon the measurement of the distortion change. For example, as shown in Fig. 14, a color elastic modulus image 30 is generated and correlated with the ROI such that the elastic modulus E and the stress $\sigma$ are displayed as numeric values. This allows the inspector to observe the color elastic modulus image 30 and to objectively diagnose the elasticity of the target portion, resulting in the determinate diagnosis. As the right view of Fig. 12 shows, for example, values of the real-time stress $\sigma$ measured with respect to the ROI and the measurement value of the elastic modulus E are plotted to form the $\sigma$-E graph 31 (dot shown in the drawing) so as to be displayed. The stress $\sigma(t)$ at the current measurement time t may be displayed by an indicator 32. The inspector is allowed to acquire the elastic modulus E of the target portion in the reference range of the distortion change ratio as the predetermined diagnostic reference or in the reference stress range for differentiating the tissue.

In the third and the fourth embodiments, it is preferable to set the reference regions R1 and R2 in the tissues such that the value of $\Delta\varepsilon2/\Delta\varepsilon1$ changes with high sensitivity in accordance with magnitude of the stress. For example, when examining the breast cancer, the reference regions may be set for the fat and the muscle, the fat and the mammary gland, and the mammary gland and the muscle. The following relationship may be established in view of the rigidity, that is, fat < mammary gland < muscle.

In the third and fourth embodiments, except the case where the compressed state is estimated using the dimension of the stress (kPa), the value of the distortion change ratio ($\Delta\varepsilon2/\Delta\varepsilon1$) may be directly used to evaluate the compressed state using the index correlated with the stress (hereinafter referred to as the stress index). In this case the elasticity data is applicable to the differentiation as the index relevant to the elasticity (hereafter referred to as the elastic index). For example, the value of the distortion change ratio ($\Delta\varepsilon2/\Delta\varepsilon1$) is directly used (see equation (14)) to use the following equation (15) instead of the stress change $\Delta\sigma$ using the elastic modulus index E (index) as replacement of the elastic modulus.

$$\texttt{E(index)} = (1/(\Delta\varepsilon2/\Delta\varepsilon1)) \times (1/\Delta\varepsilon\texttt{I}) \quad (15)$$

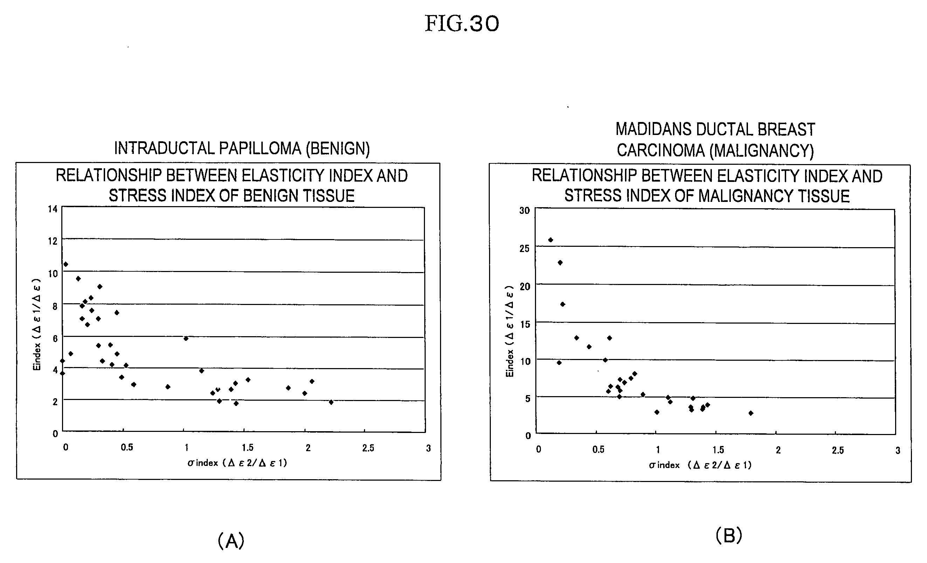

Referring to Fig. 30, the distortion change ratio ($\Delta\varepsilon2/\Delta\varepsilon1$) is directly used as the stress index σindex to explain the experimental results which allow the differentiation with respect to benign/malignancy with high accuracy using the elastic modulus index E(index). In the experiment, the reference regions R1 and R2 were set in the fat and muscle, respectively, and the target region ROI was set in the diagnosis site. The distortion changes measured in the respective regions were set to $\Delta\varepsilon1$, $\Delta\varepsilon2$ and $\Delta\varepsilon$I. The following equation is established in accordance with the third and the fourth embodiments.

$$\texttt{stress index } \sigma\texttt{index} = \Delta\varepsilon2/\Delta\varepsilon1,$$

and the following equation is further established.

$$\texttt{elasticity index Eindex} = \Delta\varepsilon1/\Delta\varepsilon\texttt{I}.$$

An example of the measurement results derived from the actual clinical trial is shown in Figs. 30(A) and (B). The drawing (A) shows the example of the intraductal papilloma (benign), and the drawing (B) shows the example of the madidans ductal breast carcinoma (malignancy).

In the case where the elasticity is indexed using the $\Delta\varepsilon1/\Delta\varepsilon$, the threshold value for differentiation with respect to benign/malignancy diagnosis is set to 5. If Eindex > 5, it is determined as being malignancy, and if Eindex ≤ 5, it is determined as being benign. This makes it possible to perform the differentiation with relatively high accuracy. However, actually the elasticity index largely depends upon the stress index as shown in Fig. 30, and accordingly, the reference stress index is required. For example, when the reference stress index range is set to establish the equation of σindex = 0.5 approximately, the elasticity index is observed that Eindex (benign) = 4, and Eindex(malignancy) = 10. Therefore, the objectivity of the differentiation may be guaranteed.

In the third and the fourth embodiments, it is assumed that the stress applied to the object in the depth direction hardly diffuses and attenuates. Actually, however, the stress attenuates in the depth direction. It is therefore preferable to calculate the elasticity data based on the stress distribution estimation process on the assumption that the stress distribution is not constant in the depth direction.

In the present and the third embodiments, the compressed state is evaluated using the distortion change ratio $\Delta\varepsilon2/\Delta\varepsilon1$. Such values as $(\Delta\varepsilon2-\Delta\varepsilon1)/\Delta\varepsilon1$ or Log($\Delta\varepsilon2/\Delta\varepsilon1$) may be used instead of the distortion change ratio $\Delta\varepsilon2/\Delta\varepsilon1$. That is, the index which allows the commonly added $\Delta\sigma$ to be erased from the correlation between $\Delta\varepsilon$ and $\Delta\sigma$ on which the stress σ - distortion ε feature of the two specific tissues are reflected may be employed to sufficiently evaluate the compressed state.

Fifth Embodiment

The third and the fourth embodiments describe the methods for evaluating the compressed state and for estimating the stress using the data of the distortion change in the two preliminarily set reference regions. In the fourth embodiment, the single broad target region L-ROI which contains the reference regions R1, R2 and the target region ROI is set. The present invention is applied to the method for obtaining the elasticity data derived from normalizing the distortion change at the respective measurement points based on the distortion change in the L-ROI.

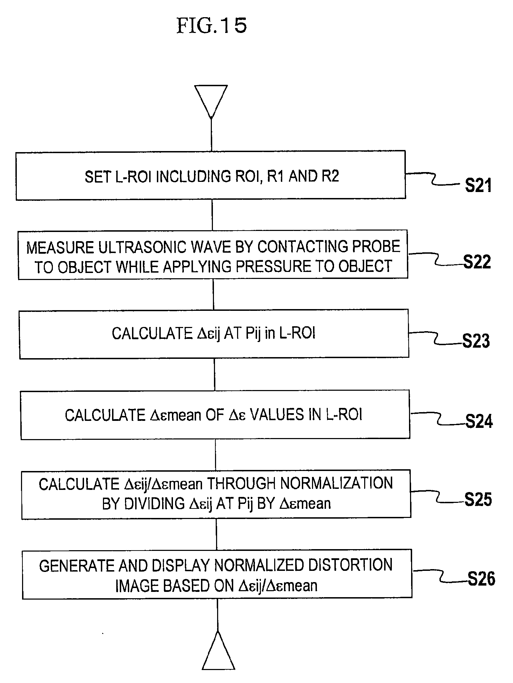

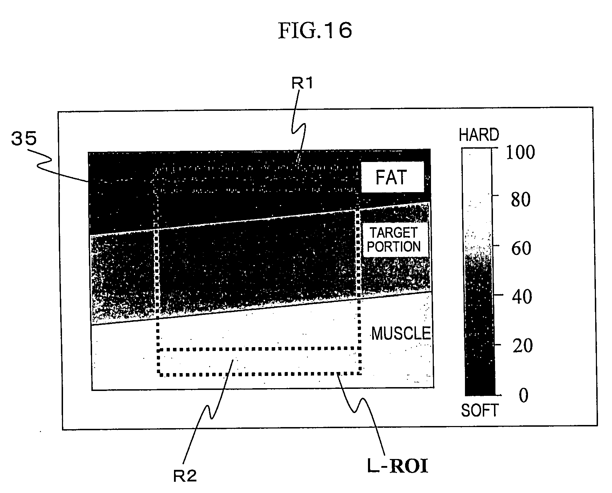

Fig. 15 is a flowchart showing the process performed in the embodiment. In step S21, the broad target region L-ROI which contains the target region ROI having the target portion such as the mammary gland, the reference region R1 having the fat portion as the normal tissue, and the reference region R2 having the muscle as the normal tissue is set on the color distortion image 35 (ultrasonic image such as B mode image or elasticity image) as shown in Fig. 16. The reference regions R1 and R2 are set in the same manner as in the third and the fourth embodiments. When the reference regions R1 and R2 have the specific positional relationship with respect to the target region ROI, for example, the reference regions R1 and R2 are layered while interposing the target region ROI in the tissue to be diagnosed as shown in Fig. 16, the reference regions R1 and R2 may be programmed to be automatically set to have the height of 5 mm by setting the broad target region L-ROI on the B mode ultrasonogram.

In step S22, the probe is brought into contact with the skin of the object 1 such that the initial compression is applied thereto (Fig. 2(B)). Then the ultrasonic wave is measured by applying the very low pressure while being changed (Fig. 2(C)). Upon measurement of the ultrasonic wave, the elasticity calculation unit 13 obtains the small distortion

change $\Delta\varepsilon$ij at each of the measurement points Pij in the entire measurement region including the broad target region L-ROI (S23). Each of the distortion changes at the measurement points P in the reference regions R1, R2, and the target region ROI is set to $\Delta\varepsilon$1, $\Delta\varepsilon$2 and $\Delta\varepsilon$I, respectively.

In step S24, the sum of values of the distortion change $\Delta\varepsilon_{ij}$ at all the measurement points Pij in the broad target region L-ROI is divided by the number of the measurement points Ntot to obtain the mean value $\Delta\varepsilon$mean of the distortion change in the broad target region L-ROI. The values of the distortion change $\Delta\varepsilon_{ij}$ at the respective measurement points are divided by the mean value $\Delta\varepsilon$mean of the distortion change in the broad target region such that the distortion change $\Delta\varepsilon_{ij}/\Delta\varepsilon$mean at the respective normalized measurement points is obtained (S25). The color scan converter 15 generates the color distortion image 35 based on the normalized distortion change $\Delta\varepsilon_{ij}/\Delta\varepsilon$mean output from the elasticity calculation unit 13 so as to be displayed on the image display unit 10 (S26). This makes it possible to index the distortion change $\Delta\varepsilon_{ij}$ at each of the measurement points P so as to be displayed.

In the aforementioned case, the normalized distortion change $\Delta\varepsilon_{ij}/\Delta\varepsilon$mean varies in accordance with the applied stress $\sigma$, and accordingly, the elasticity of the target portion cannot be evaluated determinately. Likewise the second embodiment, the distortion change ratio $\Delta\varepsilon2/\Delta\varepsilon1$ is obtained to evaluate the magnitude of the stress, based on which the elasticity of the target site on the generated elasticity image may be determinately evaluated.

Likewise the fourth embodiment, the feature curve 23 of the $\sigma$-$\Delta\varepsilon2/\Delta\varepsilon1$ shown in Fig. 13 is preliminarily measured, and the correlation between the $\Delta\varepsilon2/\Delta\varepsilon1$ of the reference regions R1 and R2, and the stress $\sigma$ is preliminarily measured, which will be stored in the memory so as to immediately estimate the stress $\sigma$ upon measurement from the measurement value of the ratio $\Delta\varepsilon2/\Delta\varepsilon1$.

Upon normalization with the mean value of the distortion change in the broad target region L-ROI, when the tissue in one of the reference regions R1 and R2 reaches the compression limit, the relationship of the distortion change between $\Delta\varepsilon1$ and $\Delta\varepsilon2$ with respect to the magnitude sharply changes. Likewise the fourth embodiment, the inspector is warned of the sharp change in the normalized distortion change ($\Delta\varepsilon2/\Delta\varepsilon$mean) or ($\Delta\varepsilon1/\Delta\varepsilon$mean) such that the compression force is adjusted to be in the predetermined range. The inspector is then allowed to diagnose the target portion while observing the color distortion image in the predetermined compressed state range.

The process as one of those for the tissue differentiation in accordance with the normalized distortion image is proposed. Specifically, when the stress is changed to be on the rise, the process is used for diagnosing whether the normalized distortion change $\Delta\varepsilon$norml of the target portion is directed on the rise or on the decline.

Specifically, in the presence of malignant tissue, the normalized distortion change $\Delta\varepsilon$norml is likely to increase accompanied with the stress increase. Meanwhile, in the presence of benign tissue, the normalized distortion change $\Delta\varepsilon$norml is likely to decrease accompanied with the stress increase. As the compressed state may be evaluated in the embodiment, the increase in the normalized distortion change $\Delta\varepsilon$norml may be quantitatively evaluated, which allows the differentiation with respect to benign-malignancy with high accuracy.

The sharp change in the normalized distortion change ($\Delta\varepsilon2/\Delta\varepsilon$mean) or ($\Delta\varepsilon1/\Delta\varepsilon$mean) at the compression limit will be described. Referring to Fig. 14, the broad target region L-ROI and the reference regions R1 and R2 are set, and the numbers of the measurement points P in the broad target region L-ROI will be set as follows.

Number of the measurement points of the tissue having the reference region R1 set: N1
Number of the measurement points of the tissue having the reference region R2 set: N2
Number of the measurement points of the tissue in the target region: N
Number of the measurement points of the tissue in the broad target region L-ROI: Ntot = N1 + N2 + N

The mean value $\Delta\varepsilon$mean of the distortion change in the L-ROI is expressed by the following equation (16).

$$\Delta\varepsilon\text{mean} = (\Sigma(\Delta\varepsilon1ij) + \Sigma(\Delta\varepsilon2ij) + \Sigma(\Delta\varepsilon ij)/\text{Ntot}$$
$$= (N1 \times \Delta\varepsilon1 + N2 \times \Delta\varepsilon2 + N \times \Delta\varepsilon)/\text{Ntot}$$
$$= \{(N1/\alpha1 + N2/\alpha2 + N/\alpha)/\text{Ntot}\} \times (\Delta\sigma/\sigma) \quad (16)$$

It is assumed that the normalized distortion change in each of the tissue regions is obtained using the same value measured in the respective tissue regions, that is, $\Delta\varepsilon1ij = \Delta\varepsilon1$, $\Delta\varepsilon2ij = \Delta\varepsilon2$, and $\Delta\varepsilon ij = \Delta\varepsilon$.

Each distortion change measured at the respective measurement points is divided by the value of $\Delta\varepsilon$mean in the equation (8) to obtain the normalized distortion change shown in the equation (17) (hereinafter referred to as the normalized distortion change $\Delta\varepsilon$norm).

$$\Delta\varepsilon norm = \Delta\varepsilon/\Delta\varepsilon mean \qquad (17)$$

The aforementioned normalization provides the index indicating how many times the value of the normalized distortion change $\Delta\varepsilon norm$ is larger than the mean value. When it is the same as the mean value, the $\Delta\varepsilon norm$ is set to "1". The normalized distortion changes $\Delta\varepsilon norml$ and $\Delta\varepsilon norm2$ in the reference regions R1 and R2 will be described with respect to two ranges, that is, the range where the compression limit is not reached and the range where the compression limit is reached, respectively.

(1) Range where the compression limit is not reached
The normalized distortion change $\Delta\varepsilon norm1$ of the fat in the reference region R1 is expressed by the following equation (18).

$$\Delta\varepsilon norm1 = \Delta\varepsilon1/\Delta\varepsilon mean$$
$$= (Ntot/\alpha1)/(N1/\alpha1 + N2/\alpha2 + N/\alpha) \qquad (18)$$

The normalized distortion change $\Delta\varepsilon norm2$ of the muscle in the reference region R2 is expressed by the following equation (19).

$$\Delta\varepsilon norm2 = \Delta\varepsilon2/\Delta\varepsilon mean$$
$$=(Ntot/\alpha2)/(N1/\alpha1 + N2/\alpha2 + N/\alpha) \qquad (19)$$

(2) Range where the compression limit is reached

The normalized distortion change $\Delta\varepsilon norml$ of the fat in the reference region R1 is expressed by the following equation (20).

$$\Delta\varepsilon norm1 = \Delta\varepsilon1/\Delta\varepsilon mean$$
$$= (Ntot/\alpha1')/(N1/\alpha1' + N2/\alpha2 + N/\alpha) \qquad (20)$$

The normalized distortion change $\Delta\varepsilon norm2$ of muscle in the reference region R2 is expressed by the following equation (21).

$$\Delta\varepsilon norm2 = \Delta\varepsilon2/\Delta\varepsilon mean$$
$$=(Ntot/\alpha2)/(N1/\alpha1' + N2/\alpha2 + N/\alpha) \qquad (21)$$

Assuming that $(\alpha1'>>\alpha2, \alpha)$, the $\Delta\varepsilon norm1$ in the equation (20) gets closer to "0", and the $\Delta\varepsilon norm2$ in the equation (21) gets closer to the value in the following equation (22).

$$(Ntot/\alpha2)/(N2/\alpha2 + N/\alpha) \qquad (22)$$

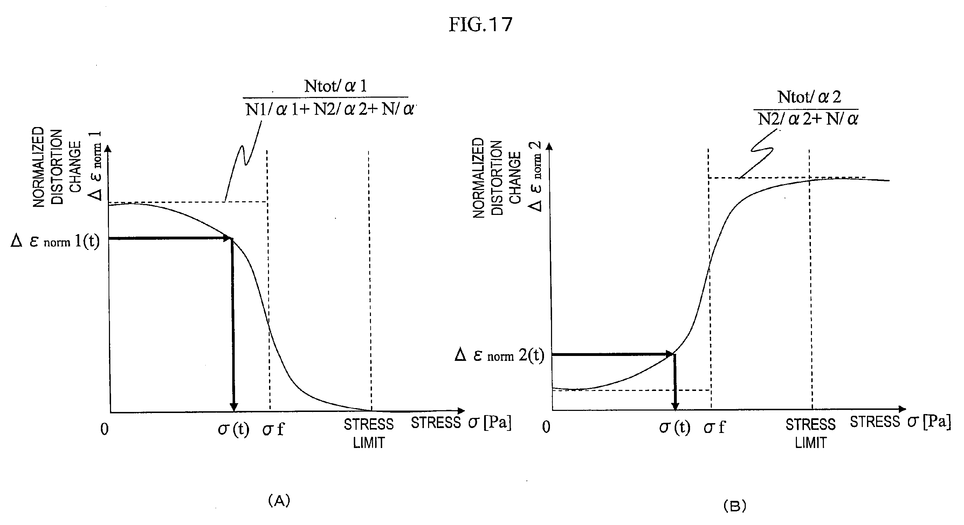

The resultant behavior is shown in Figs. 17(A) and 17(B). As Fig. 17 clearly shows, when the fat tissue exceeds the boundary stress $\sigma f$ in the compression limit region, the normalized distortion change $\Delta\varepsilon norm1$ of the fat sharply decreases to be close to zero, and the normalized distortion change $\Delta\varepsilon norm2$ of the muscle sharply increases to be converged to a constant value of the equation (22). In the actual tissue, the behavior transition is considered to be continuous rather than discontinuous around the boundary of the compression limit, resulting in the continuous response as shown in the drawing.

The use of the behavior allows estimation of the common stress depending on the resultant value. Likewise the third embodiment, for example, the ratio of the normalized distortion change between Δεnorm1(t) and Δεnorm2(t) is primarily correlated with the stress σ(t) as the index of the compressed state. The elasticity data such as the elastic modulus E(t) is calculated real-time using the stress σ from the distortion change Δε(t) in the target region to display the obtained data in such form as the numeric value, graph and indicator. The stress is divided by the distortion to obtain Young's modulus as the elastic modulus E(t). The Young's modulus in the target region is comprehensively derived from the following equation (23).

$$E(t) = \Delta\sigma(t)/\Delta\varepsilon(t) \quad (23)$$

Not only the mean value but also the median value may be used for the normalization. The statistical information such as fitting with the normal distribution may be used. The region where the average distortion change is measured (mammary gland in the embodiment) may be automatically recognized in the single broad target region so as to generate the normalized distortion based on the distortion change in the region.

Sixth Embodiment

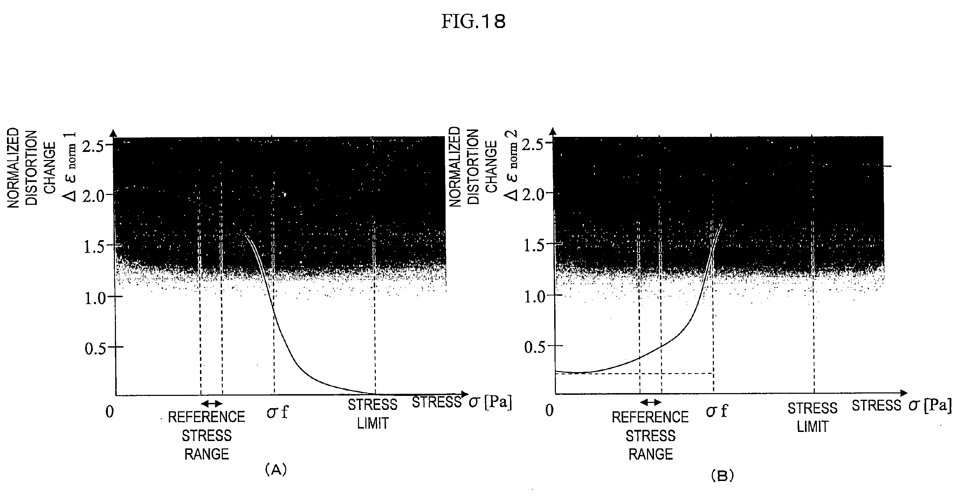

In the embodiment, the reference stress is determined using the normalized distortion image with gradation based on the information with respect to the normalized distortion change derived from the fifth embodiment. In the embodiment the hue is allocated in accordance with the color map shown in Figs. 18(A) and 18(B) in accordance with the level of the normalized distortion to form the normalized distortion image using the normalized distortion change. For example, the hue is allocated as follows to form the color map with continuous gradation applied to the in-between spaces.

    normalized distortion change of around 0.0 → blue
    normalized distortion change of around 1.0 → green
    normalized distortion change of 2.0 or larger → red

The aforementioned allotment in the color map allows the hue around the boundary stress σf both in the fat and muscle portions to be sharply changed. Additionally, the boundary at which the hue changes may also be set around the predetermined reference stress range. For example, in the fat portion, the hue may be set to be turned from red to orange at a portion around the reference stress range. Likewise the muscle portion, the hue may be set to be turned from blue to light blue at a portion around the reference stress range.

Alternatively, the hue of the normalized distortion image in the tissue region selected as the reference region, for example, fat or muscle which distributes inside the broad target region may be formed. Specifically, the transition of the hue in the reference region to the one corresponding to the reference stress range is sensed, and the resultant elasticity data in the target region at the timing is used for the differentiation. The compressed state just before the hue transition from blue to green of the muscle portion on the normalized distortion image is determined as being appropriate. The elasticity image of the target region in the appropriate compressed state may be used for the diagnosis.

At the time when the measurement is performed in the reference range of the predetermined normalized distortion range as the stress criterion, the measurement point in the reference region may be provided with a hue of purple. This makes it possible to clearly show that the measurement point is in the reference range of the normalized distortion change.

The embodiment will be described in more details using specific conditions.

It is assumed that each number of the measurement points is the same in the reference regions R1 and R2, and the target region ROI, and the measurement points are set as follows:

    Number of the measurement points of the tissue having the reference region R1 set: N
    Number of the measurement points of the tissue having the reference region R2 set: N
    Number of the measurement points of the tissue in the target region ROI: N
    Number of the measurement points of the tissue in the broad target region L-ROI: Ntot = 3N

It is further assumed that values of α1, α2, and α each indicating the non-linearity in the respective tissues are set to values as follows.

$\alpha1 = 1$
$\alpha2 = 8$
$\alpha = 16$

The equations (18), (19) and (22) are calculated using the aforementioned values as follows.

$$(Ntot/\alpha1)/(N1/\alpha1 + N2/\alpha2 + N/\alpha) = 2.5$$

$$(Ntot/\alpha2)/(N1/\alpha1 + N2/\alpha2 + N/\alpha) = 0.3$$

$$(Ntot/\alpha2)/(N2/\alpha2 + N/\alpha) = 2.0$$

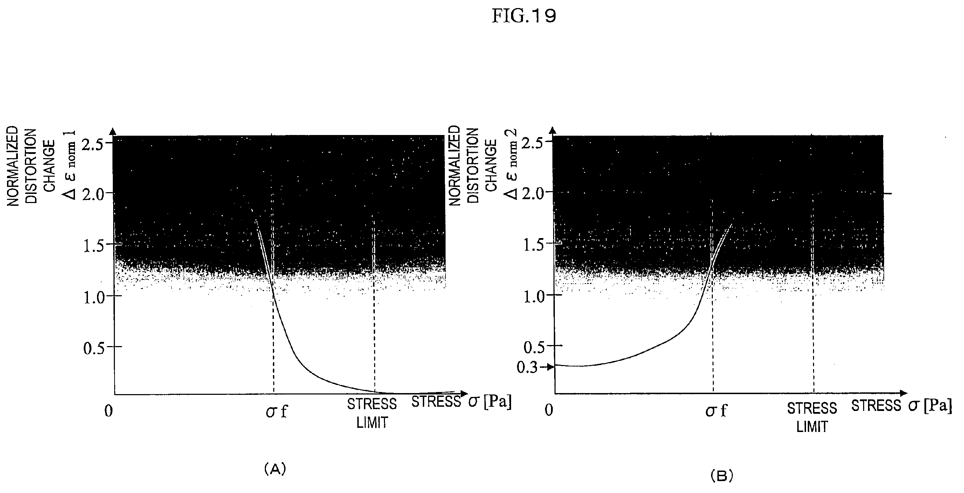

The resultant normalized distortion changes $\Delta\varepsilon$norm1 and $\Delta\varepsilon$norm2 have behaviors as shown in Figs. 19(A) and 19(B). Referring to FIG. 19(A), the normalized distortion change $\Delta\varepsilon$norm1 takes the value around 2.5 in the range where the fat tissue in the reference region R1 does not reach the compression limit, and the normalized distortion image in the region is colored in red. When it exceeds the boundary stress $\sigma f$ to define the boundary of the compression limit of the fat tissue, the normalized distortion change $\Delta\varepsilon$norm1 of the fat portion sharply decreases to get close to zero. The color then turns into blue. Meanwhile, as shown in Fig. 19(B), in the range where the fat portion in the reference region R1 does not reach the compression limit, the normalized distortion change $\Delta\varepsilon$norm2 of the muscle region in the reference region R2 takes the value around 0.3. The normalized distortion image in the muscle region is colored in blue. However, when it exceeds the boundary stress $\sigma f$ to define the boundary of the compression limit in the fat tissue, the normalized distortion change $\Delta\varepsilon$norm2 of the muscle sharply increases to get close to 2.0. The color is turned into red.

Seventh Embodiment

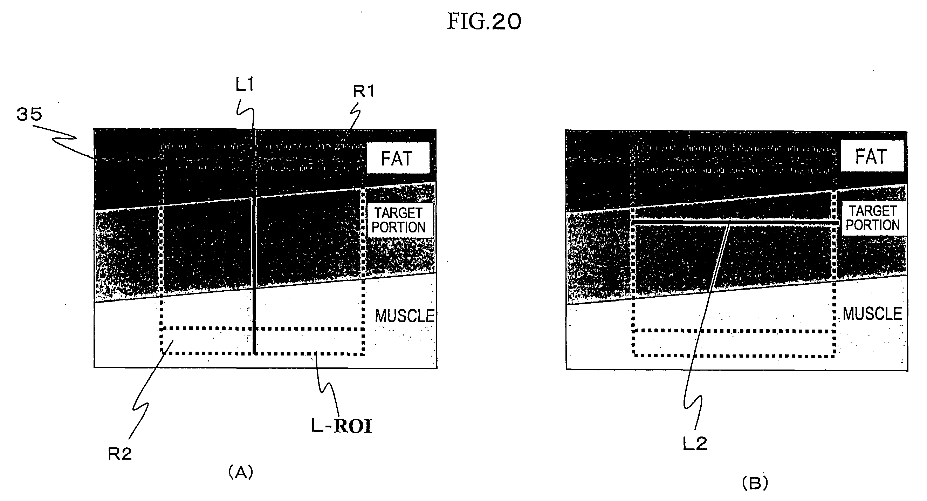

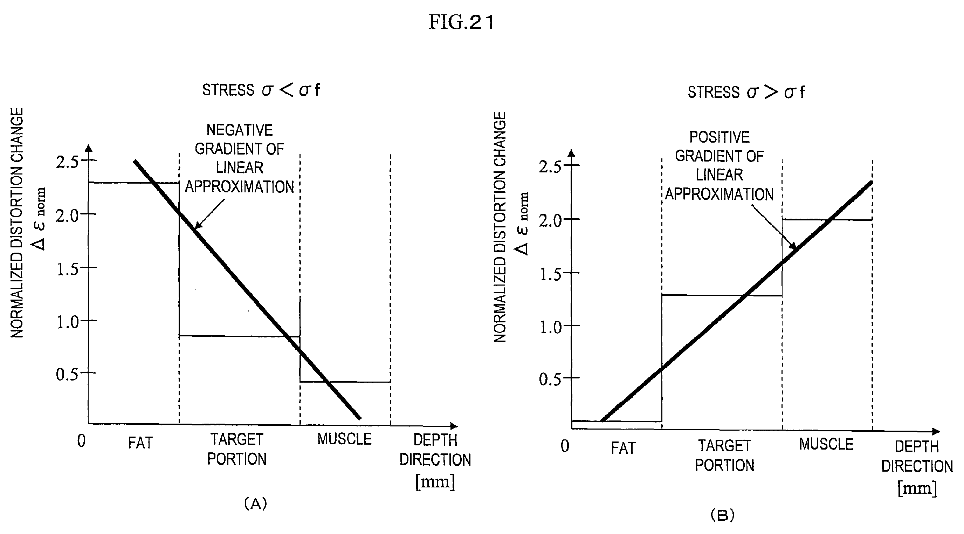

As described in the fifth embodiment, as the amount of compression is intensified, the normalized distortion change $\Delta\varepsilon$norm1 of the adjacent fat portion changes in the decreasing direction, and the normalized distortion change $\Delta\varepsilon$norm2 of the muscle in the depth portion changes in the increasing direction. Then the distribution of the normalized distortion change in the depth direction shown in Fig. 20 is focused. Referring to Fig. 20(A), a focus line L1 is set such that the distribution of the normalized distortion change thereabove is observed. As shown in FIGS. 21(A) and 21(B), as the amount of compression is intensified direction of the gradient of the distortion change distribution toward the depth direction (for example, the gradient in the depth direction approximated with the linear function) is changed from negative to the positive. The reference range of the appropriate distortion change distribution gradient is set in reference to the gradient value of the distortion change distribution in the depth direction so as to determine the reference stress range.
Referring to Fig. 20(B), another focus line L2 is set, and the mean value of the normalized distortion change at the measurement points at the same depth such that the normalized distortion change distribution in one depth direction may be evaluated. The level of the stress may be roughly obtained by displaying the numeric value as the aforementioned gradient. In the embodiment, the reference regions R1 and R2 do not have to be set, thus simplifying the process.

Eighth Embodiment

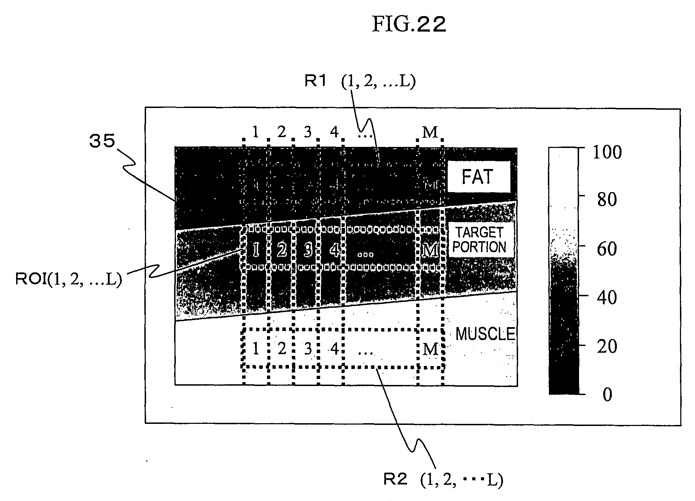

In the embodiment, the reference regions R1, R2 and the target region ROI are divided into a plurality of line regions (1, 2,..., M) with respect to the same measurement line as shown in Fig. 22 such that the distortion change in the reference regions R1, R2 and the target region ROI for each line region is obtained. The distribution of the distortion change in the line region (that is, the distribution of the distortion change in the depth direction) is obtained to evaluate the compressed state in the same way as in the seventh embodiment. The line region may be set as the one derived from binding a plurality of actual lines of the ultrasonic beam radiated from the probe.

Ninth Embodiment

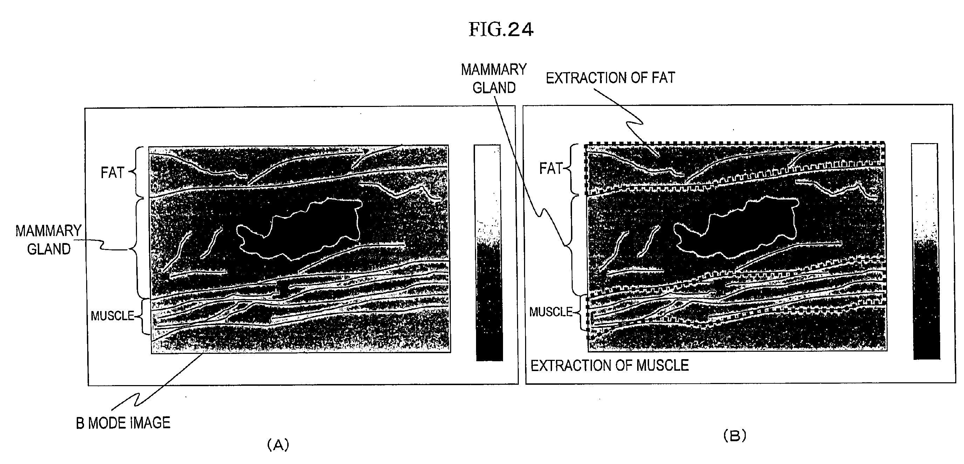

The elastic modulus (Young's modulus) of the target region is comprehensively obtained through the equation of $E(t) = \Delta\sigma(t)/\Delta\varepsilon(t)$ in the equation (23) of the fifth embodiment. The equation may be applied independently for the respective line regions shown in Fig. 24. Assuming that the coordinate of each of the line regions is set to i, and the coordinate in the depth direction is set to j in the ninth embodiment, the elastic modulus $E_{ij}(t)$ at each of the measurement points may be obtained through the following equation (24) using the stress change $\Delta\sigma_i(t)$ for each line region and the distortion change $\Delta\varepsilon_{ij}$ at each of the respective measurement points.

$$E_{ij}(t) = \Delta\sigma_i(t)/\Delta\varepsilon_{ij}(t) \qquad (24)$$

In other words, the elasticity modulus at each of the measurement points may be obtained. As the elastic modulus of the target region may be obtained in the form of distribution, the embodiment provides and displays the Young's modulus image having the level graded as the elasticity image in consideration with the difference in the stress distribution in the line direction.

In the embodiment, it is assumed that the stress applied to the respective line regions in the depth direction is kept constant. Actually, however, the stress distribution may not be constant in the depth direction. In this case, it is preferable to calculate the elasticity data based on the known stress distribution estimation process.

The depth of the muscle portion is obtained from the position of the reference region set in the muscle. The reference stress range may be changed to an appropriate one depending on the depth of the muscle.

Tenth Embodiment

The aforementioned embodiments show the process for quantitatively evaluating the compressed state in the ultrasonic diagnostic apparatus without pressure sensor. A certain patient cannot have sufficient area of the normal tissue (for example, fat) where the reference regions R1 and R2 are set in the measurement section. In such a case, the voice coupler with the known stress-distortion feature (for example, SONAR-AID and SONAGEL) as disclosed in JP 2005-66041 A is interposed between the skin of the object and the probe, and allowed to serve as being one of the reference regions.

Eleventh Embodiment

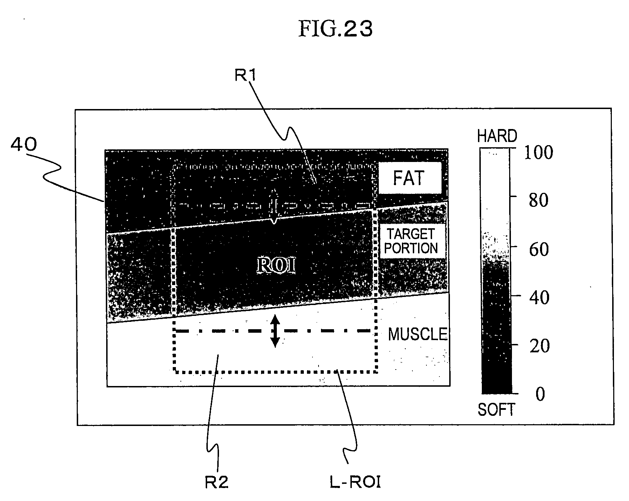

In the embodiment, an example for setting the broad target region L-ROI, and the reference regions R1 and R2 will be described referring to Fig. 23. As shown in the drawing, the reference regions R1 and R2 are set inside the broad target region L-ROI. The inspector is allowed to operate such input device as a track ball to set the reference regions R1 and R2 in the fat portion and the muscle portion through the control interface 17. For example, the bottom line of the reference region R1 set in the fat portion may be adjusted up and down. Likewise the top line of the reference region R2 set in the muscle portion may be adjusted up and down.

Twelfth Embodiment

In the embodiment, the reference regions R1 and R2 are automatically set. When breast cancer is intended to be examined, the ultrasonic wave will be blocked by the rib below the muscle portion, and accordingly, the area from the rib surface to the depth portion becomes a non-echo region where the reflection echo signal cannot be obtained. The boundary between the non-echo region and the echo region is detected through the threshold processing so as to detect the boundary between the muscle and the rib surface.

As disclosed in JP 2005-118152 A, the error in the elasticity data with respect to the non-echo region which locates deeper than the rib surface is detected to locate the rib region such that the boundary between the muscle and the rib is detected.

Referring to Figs. 24(A) and 24(B), the template which reflects arrangement, the configuration, the ultrasonic echo level, and pattern of the ultrasonic echo level (for example, fiber pattern, speckle pattern) of the tissues at the object site for examination of the breast cancer is formed. The regions of the fat, mammary gland, tumor, muscle and rib may be automatically recognized through matching with the template. This makes it possible to automatically set the reference regions and the target region independently. Not only the reflection echo signals but also the elasticity distribution image such as the distortion image may be objected to the threshold processing so as to detect the boundary among the tissues, and the outline. On the template, each layer region of the fat, mammary gland, muscle is divided and displayed on the B mode area in the translucent manner. The respective regions may be determined by performing the fine adjustment on the B mode image with the mouse.

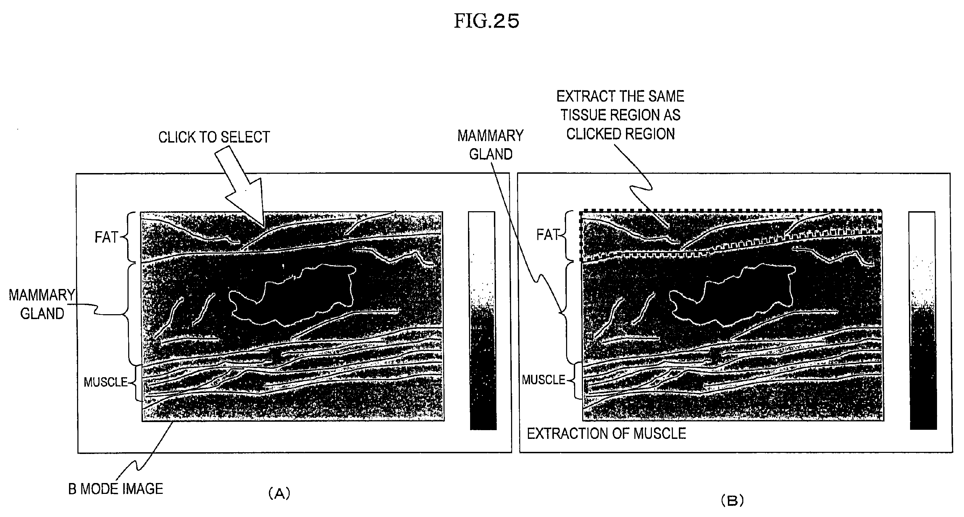

Furthermore, it is not fully-automatic recognizing, as shown in Fig. 25(A), when the inspector selects the representative point of the tissue (for example, fat portion) with the reference region set on the ultrasonic image by clicking the pointer of the control interface 17, the same tissue region as the one shown by the dotted line in the drawing is extracted from the point of clicking to the periphery such that the reference region with the required size is automatically set. As the measurement points of the reference region may be maximized to increase the accuracy, and the dependency on the inspector is reduced by automatically setting the reference regions.

Thirteenth Embodiment

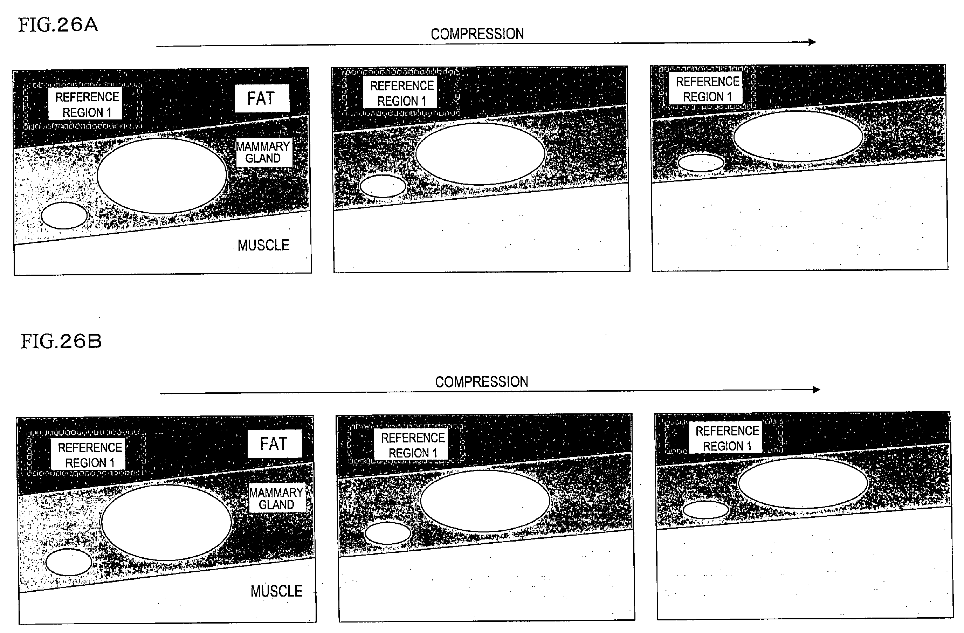

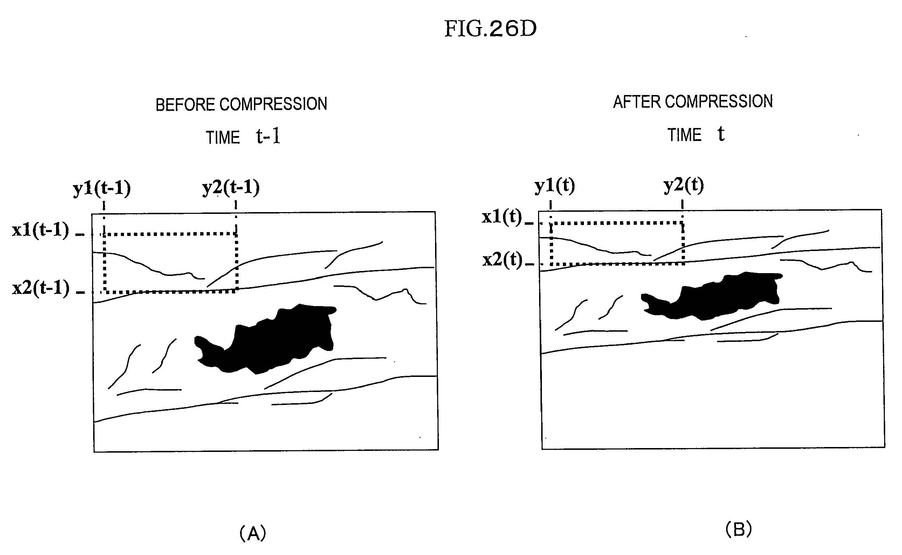

Figs. 26A and 26B show the embodiment having the reference region moved and modified to follow the tissue deformed under the compression. Referring to Fig. 26A, as the fat portion with the reference region R1 is likely to be deformed under the compression, the thickness of the fat portion is reduced as the compression force is intensified from the left to the right side in the drawing. Then the fat portion deviates from a portion of the reference region R1, which causes the mammary gland tissue to be mixed. As a result, the measurement error occurs. In the embodiment, the reference region R1 is moved and deformed to follow the deformed fat portion. The data with respect to the distortion change from regions of the respective tissues may be used at maximum measurement points, thus allowing the stress evaluation with high accuracy.

The aforementioned follow-up process of the reference region may be performed using the displacement data at the respective measurement points. The function for automatically recognizing the tissue as described in the twelfth embodiment may be used to follow the same tissue.

Fig. 26B shows an example using the reference region R1. Likewise, the reference regions R2, the target region ROI, and the broad target region L-ROI are moved and deformed to follow the deformed tissue as shown in Fig. 26B.

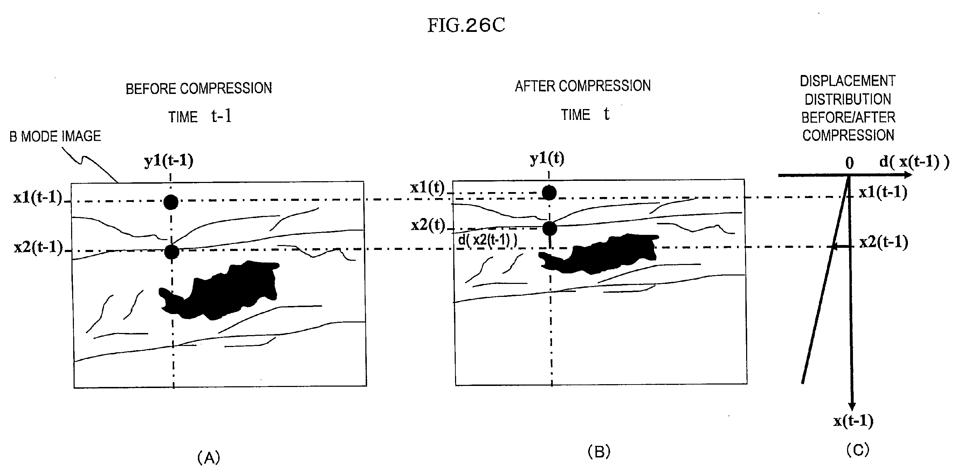

The specific method for performing the follow-up process of the reference region R1 will be described. The process for obtaining the displacement distribution (displacement frame data) performed by the displacement measurement unit 12 shown in Fig. 1 will be described referring to Fig. 26C. With the coordinate system having the y-coordinate in the horizontal direction, and x-coordinate in the depth direction, two measurement points along the line y1, that is, $(x1, y1)$, $(x2, y1)$ are focused. It is assumed that the tissue is compressed at the transition time from t-1(past) to t(present). The tissue at the coordinates of $(x1(t-1), y1(t-1))$ at the time of t-1 is moved to the coordinate of $(x1(t), y1(t))$ at the time of t. Likewise, the tissue at the coordinate with $(x2(t-1), y1(t-1)$ at the time of t-1 is moved to the coordinate $(x2[t], y1[t])$ at the time of t.

The displacement measurement unit 12 calculates the displacement $d(x,y)$ at all the measurement points $[x, y]$. For example, the displacement distribution $d(x(t-1))$ along the line y1 from the time t-1 to t is obtained as shown in the drawings. The x coordinates x1(t) and x2(t) are derived from the following equations.

$$x1(t) = x1(t-1) + d(x1(t-1))$$

$$x2(t) = x2(t-1) + d(x2(t-1))$$

Likewise, the y coordinates y1(t) and y2(t) may be obtained. With the aforementioned process, when the rectangular reference region R1 which contains the following four points is set at the time t-1, the movement of the tissue defined by the four points may be detected as shown in Fig. 26D. The tissue in the region R1 is designated as the same tissue region.

$$(x1(t-1), y1(t-1))$$

$$(x2(t-1), y1(t-1))$$

$$(x1(t-1), y2(t-1))$$

$$(x2(t-1), y2(t-1))$$

The aforementioned process may be performed real-time, and the same tissue region as the reference region R1 may be tracked to follow as shown in Fig. 26B.

In the aforementioned description, the reference region R1 is objected to the follow-up process using the displacement data with respect to the four-point coordinates arranged at corners which define the reference region R1. Besides the aforementioned process, the follow-up process may be performed with high accuracy based on more data with respect to the displacement at the coordinates arranged along the line as the boundary of the reference region R1.

The data of the displacement at the inner coordinate of the reference region R1 may be used to perform the follow-up process with higher accuracy.

Fourteenth Embodiment

In the aforementioned embodiments, it is assumed that the elasticity image is generated real-time. Actually, in the process for following (tracking) the reference region such as the muscle real-time, when the cross section of the measurement is changed, the muscle position is also largely changed, thus bothering the inspector to set the reference region again. In the embodiment, the size and the position of the reference region and the target region may be set on the still image after freezing. Alternatively, the size and the position of the set reference region and the target region may be adjusted.

The reference region and the target region may be set on the still image after freezing rather than setting during the real-time measurement so as to confirm whether the compressed state at the timing is appropriate.

Fifteenth Embodiment

Likewise the elasticity data (including elasticity index) of the target region obtained based on the aforementioned embodiment, the stress data (including stress index) may be obtained as the data with respect to the compressed state. The specific process for performing the tissue differentiation using the aforementioned data will be described.

It is preferable to select at least the muscle for one of the reference regions R1 and R2. In the actual measurement of the elasticity, the muscle portion may be included on the ultrasonogram of substantially all the mammary gland, and is vertically arranged with respect to the compression direction. So the data with respect to the compression may be correctly detected. Meanwhile, the thickness of the fat portion differs depending on the individual, and it is often the case that the fat with the appropriate thickness hardly exists on the upper surface of the target region. As the distance target region inside the mammary gland is closer to the muscle than the fat portion, the stress applied to the target region may be estimated with higher accuracy. As the rib below the muscle serves as the fixed end, the distortion data is unlikely to be influenced by the difference in the rigidity or configuration of the peripheral tissue. This makes it possible to estimate the stress under the common condition for different objects, resulting in high reproducibility and objectivity in the stress estimation.

Sixteenth Embodiment

In the embodiment, a specific example for differentiation with respect to benign/malignancy of the tumor will be described. It is known that the normalized distortion change image by itself is advantageous to differentiate with respect to benign/malignancy of the tumor. In the fourth and the fifth embodiments, the present invention is applied to the normalized distortion change image. For example, in the sate just before the transition of the hue corresponding to the muscle from blue to green, it is determined that the condition for the reference stress range is satisfied. Then the normalized distortion change image of the target region derived in the compressed state is selected to allow the quantitative differentiation of the elasticity of the target region.

In the tissue differentiation using the normalized distortion change image, the elasticity scoring technique may be applied for evaluating the elasticity of the target site with the score based on the relative relationship between the region with the low echo signal of the B mode image and the rigid region of the normalized distortion change image. The diagnostic method using the elasticity scoring is disclosed in WO2005-025425.

Seventeenth Embodiment

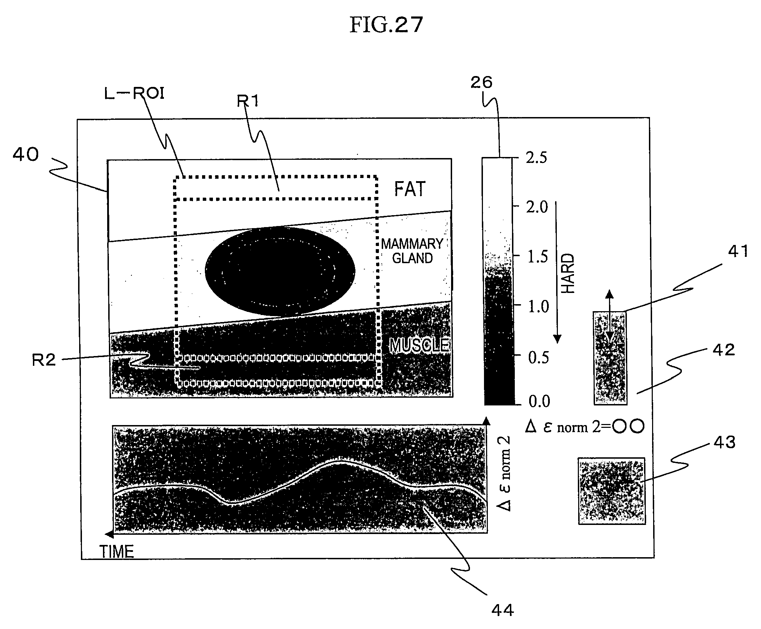

Fig. 27 shows a preferred embodiment of the elasticity image displayed by the ultrasonic diagnostic apparatus according to the present invention. The sharp change in the normalized distortion change $\Delta\varepsilon norm1(t)$ or $\Delta\varepsilon norm2(t)$ in the reference region R1 or R2 is detected using the normalized distortion change image as described in the fifth and the sixth embodiments so as to determine adequacy of the reference stress. In this case, it is determined that the state just before the transition of the hue corresponding to the muscle from blue to green is in the reference stress range. Such determination, however, tends to be made by the inspector's objectivity.

In the embodiment, in order to evaluate the sharp change in the $\Delta\varepsilon norm1(t)$ or the $\Delta\varepsilon norm2(t)$ objectively, it is preferable to display the bar gauge 41 with its length changed by the normalized distortion change $\Delta\varepsilon norm2(t)$ in the muscle as the stress index corresponding to the normalized distortion change image 40 as the elasticity image in Fig.

27 shows. In this case, the mean value of the Δεnorm2(t) may be displayed by the numeric value 42 on the screen. The hue of the Δεnomr2(t) in the reference region R2 may also be displayed on the indicator 43. The change in the Δεnorm2(t) as the stress index during the measurement may be displayed with a graph 44 like the oscilloscope.

The embodiment allows the visual evaluation with respect to the compressed state at the current measurement time. The distortion change ratio (Δε2/Δε1) as described in the second and the third embodiments may be employed as the stress index. The stress σ(t) estimated based on the stress index may be employed.

Eighteenth Embodiment

Referring to Fig. 27, it is determined whether or not the compressed state at the current measurement time is in the predetermined reference stress range based on the stress index estimated by the present invention. In the embodiment, when the stress index exceeds the predetermined referenced stress range, the warning is displayed or the inspector is warned by the warning tone, thus avoiding the misdiagnosis owing to the one under the excessive compression.

The arrow image may be displayed to guide whether the reference stress range is in the pressure-increasing direction from the current compressed state, or in the pressure-decreasing direction. The hue in the reference region may be changed or illuminated so as to be recognized at the timing when the condition for the reference stress range is satisfied. The elasticity image may be displayed only when the condition for the stress reference range is satisfied. The state where the pressure is getting close to the appropriate level may be seen on the image on the display by values of the luminance of the elasticity image and translucence permeation rate, which changes as the reference stress range is approaching. The aforementioned method for displaying the translucent elasticity image is disclosed in WO2004-039262. The elasticity image obtained in the optimum compressed state in the center of the stress reference range may be automatically selected and fed to the inspector.

Nineteenth Embodiment

Fig. 14 shows an example where the relationship between the elastic modulus and the stress is plotted into the graph to be displayed. The relationship between the magnitude of the distortion in the target region and the stress may be formed into the graph.

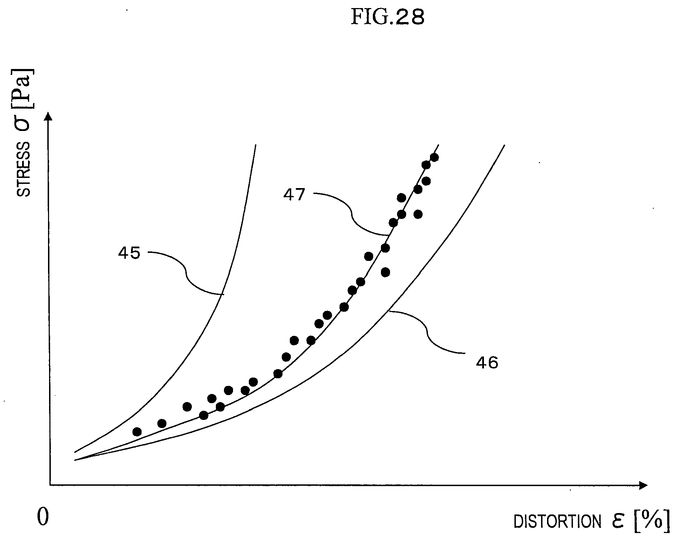

In the embodiment, the stress-distortion relationship in the target region ROI is real-time plotted on the graph to be displayed as shown in Fig. 28. Each stress σ-distortion ε relationship of the tissue (fat, mammary gland, muscle, fibrous adenoma, ductal breast carcinoma, madidans ductal breast carcinoma) may be formed into the graph. The graph shows the σ-ε relationship 45 of the breast ductal carcinoma, the σ-ε relationship 46 of the fibrous adenoma, and the currently measured σ-ε relationship 47, respectively. In this case, the respective tissues may be displayed in different colors. The similarity-based comparison between the stress-distortion curve of the target region in the current diagnosis and any one of the curves may be made real-time. In the drawing, as the curve of the stress-distortion relationship graph during the measurement is similar to that of the fibrous adenoma, it is determined as the fibrous adenoma.

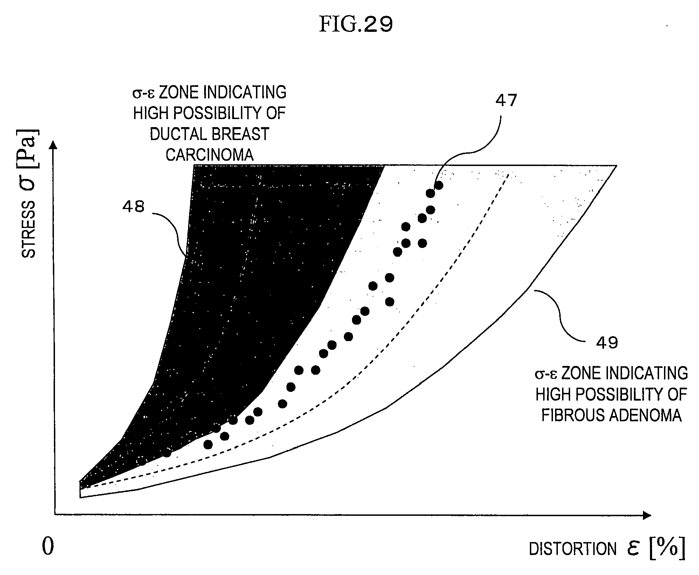

Referring to Fig. 29, stress-distortion zones 48 and 49 are provided where the determination is made with respect to the tissues. This makes it possible to determine the zone in which the currently measured stress-distortion relationship 47 exists. The specific data is allocated to each of the tissue zones 48 and 49. In the case where the data relates to the hue, the hue allocated to the zone in which the currently measured stress-distortion relationship 47 exists is added to the target region so as to be easily identified by the inspector.

In this case, the determination to select the curve with the similarity may be automatically performed through calculation of the correlation between the curves. Alternatively, the determination whether the target tissue is the FA (fibrous adenoma), the DCIS (ductal breast carcinoma), or DC (madidans ductal breast carcinoma) may be automatically made based on the value of the non-linear parameter α showing the non-linearity which is automatically calculated.

Likewise the present embodiment, the elastic modulus-stress curve and the zone which reflect features of the tissues may be displayed so as to allow the determination to be made by performing the same process.

The embodiment where the curve relevant to the compressed state such as the distortion-stress and elastic modulus-stress may be displayed is not limited to the process for estimating the compressed state. For example, the use of the pressure sensor for measuring the stress is also applicable.

Having been described with respect to the embodiment where the present invention is applied to the mammary gland region. However, it is not limited to the one as described above, and is applicable to the other tissue such as prostatic gland and the thyroid gland. Especially, the present invention is applicable as the stress indicator by setting the tissue such as the fat and muscle in the tapetum of the prostatic gland as the reference region.

The method for evaluating the compressed state is not limited to the elasticity image diagnosis, but is applicable to the Doppler image diagnosis for evaluating the bloodstream data. It is well known that the result of the Doppler image diagnosis may vary depending on the compressed state. Accordingly, the method for evaluating the compressed state according to the present invention may be employed simultaneously with the use of the Doppler image diagnosis.

**Claims**

1. An ultrasonic diagnostic apparatus comprising:

    an elasticity calculation unit (13) for calculating elasticity data of a tissue at plural measurement points based on a pair of frame data of reflection echo signals each measured at a different time, which are obtained in a process where a compressed state of an object is changed;
    an elasticity image generation unit for generating an elasticity image based on the elasticity data calculated by the elasticity calculation unit so as to be displayed on a display unit (10); and
    a compressed state evaluation unit (19) for evaluating the compressed state based on distortion change data of the tissue at the measurement point, wherein the evaluated compressed state correlated with the elasticity image is displayed on the display unit,
    wherein the compressed state evaluation unit (19) is configured to evaluate the compressed state based on a cumulative value ($=\sum\Delta\varepsilon$) of the distortion change of the tissue at the measurement point,
    **characterized in that**
    the compression state evaluation unit (19) is configured to acquire the distortion change $\Delta\varepsilon$ of the tissue at the measurement point in a reference region set in a specific tissue, and to obtain the cumulative distortion $\varepsilon$ ($=\sum\Delta\varepsilon$) of the specific tissue based on the distortion change $\Delta\varepsilon$, and a stress $\sigma$ corresponding to the cumulative distortion $\varepsilon$ based on a preliminarily measured and stored stress-distortion feature of the specific tissue so as to evaluate the compressed state based on the obtained stress $\sigma$.

2. The ultrasonic diagnostic apparatus according to claim 1, wherein the compressed state evaluation unit (19) is configured to acquire the distortion change $\Delta\varepsilon$ of the tissue in a set target region from the elasticity calculation unit (13), and to obtain a cumulative distortion $\varepsilon$ by accumulating values of the distortion change $\Delta\varepsilon$ from a state where the compression state is zero.

3. The ultrasonic diagnostic apparatus according to claim 1, wherein:

    the compressed state evaluation unit (19) is configured to output the obtained stress $\sigma$ to the elasticity calculation unit (13); and
    the elasticity calculation unit (13) is configured to obtain the elasticity data including at least an elastic modulus with respect to the set target region based on the distortion change $\Delta\varepsilon$ and the stress $\sigma$.

4. The ultrasonic diagnostic apparatus according to claim 1, wherein the compressed state evaluation unit (19) is configured to evaluate the compressed state based on a relationship of the distortion change corresponding to two reference regions set in two different tissues.

5. The ultrasonic diagnostic apparatus according to claim 4, wherein the compressed state evaluation unit (19) is configured to evaluate the compressed state based on a ratio of the distortion change ($=\varepsilon1/\varepsilon2$ or $\varepsilon2/\varepsilon1$) corresponding to the two reference regions.

6. The ultrasonic diagnostic apparatus according to claim 4, wherein the compressed state evaluation unit (19) is configured to obtain the compressed state corresponding to the ratio of the distortion change based on a relationship between a ratio $\Delta\varepsilon2/\Delta\varepsilon1$ of the distortion changes in the two tissues and the compressed state, which is preliminarily measured and stored.

7. The ultrasonic diagnostic apparatus according to claim 3, wherein the elasticity image generation unit generates an image of the stress or the compressed state obtained by the compressed state evaluation unit (19) using at least one of a numeric value, a bar chart, and a graph.

8. The ultrasonic diagnostic apparatus according to any one of claims 4 to 6, wherein the tissue having the reference region set therein is at least a muscle.

**Patentansprüche**

1. Ultraschalldiagnosevorrichtung, die Folgendes umfasst:

   eine Elastizitätsberechnungseinheit (13) zum Berechnen von Elastizitätsdaten eines Gewebes an mehreren Messpunkten basierend auf einem Paar Rahmendaten von Reflexionsechosignalen, die jeweils zu einer unterschiedlichen Zeit gemessen wurden, die bei einem Verfahren erhalten werden, in dem der Kompressionszustand eines Objekts verändert wird;
   eine Elastizitätsbild-Erzeugungseinheit zum Erzeugen eines Elastizitätsbilds basierend auf den Elastizitätsdaten, die durch die Elastizitätsberechnungseinheit berechnet werden, um dieses auf einer Anzeigeeinheit (10) anzuzeigen; und
   eine Kompressionszustand-Bewertungseinheit (19) zum Bewerten des Kompressionszustands basierend auf Verformungsänderungsdaten des Gewebes bei dem Messpunkt, wobei der bewertete Kompressionszustand, der mit dem Elastizitätsbild korreliert wird, auf der Anzeigeeinheit angezeigt wird,
   wobei die Kompressionszustand-Bewertungseinheit (19) konfiguriert ist, den Kompressionszustand basierend auf einem kumulativen Wert (= $\sum\Delta\varepsilon$) der Verformungsänderung des Gewebes bei dem Messpunkt zu bewerten, **dadurch gekennzeichnet, dass**
   die Kompressionszustand-Bewertungseinheit (19) konfiguriert ist, die Verformungsänderung $\Delta\varepsilon$ des Gewebes bei dem Messpunkt in einem Bezugsbereich, der in einem bestimmten Gewebe festgelegt wurde, zu erfassen, und die kumulative Verformung $\varepsilon\,\square(=\sum\Delta\varepsilon)$ des bestimmten Gewebes basierend auf der Verformungsänderung $\Delta\varepsilon$ und eine Belastung $\sigma$, die der kumulativen Verformung $\varepsilon$ entspricht, basierend auf einem vorher gemessenen und gespeicherten Belastungsverformungsmerkmal des bestimmten Gewebes zu erhalten, um so den Kompressionszustand basierend auf der erhaltenen Belastung $\sigma$ zu bewerten.

2. Ultraschalldiagnosevorrichtung nach Anspruch 1, wobei die Kompressionszustand-Bewertungseinheit (19) konfiguriert ist, die Verformungsänderung $\Delta\varepsilon$ des Gewebes in einem festgelegten Zielbereich von der Elastizitätsberechnungseinheit (13) zu erfassen und eine kumulative Verformung $\varepsilon$ durch Aufsummieren von Werten der Verformungsänderung $\Delta\varepsilon$ von einem Zustand, in dem der Kompressionszustand Null ist, zu erhalten.

3. Ultraschalldiagnosevorrichtung nach Anspruch 1, wobei:

   die Kompressionszustand-Bewertungseinheit (19) konfiguriert ist, die erhaltene Belastung $\sigma$ an die Elastizitätsberechnungseinheit (13) auszugeben; und
   die Elastizitätsberechnungseinheit (13) konfiguriert ist, die Elastizitätsdaten zu erhalten, die wenigstens einen Elastizitätsmodul in Bezug auf den festgelegten Zielbereich basierend auf der Verformungsänderung $\Delta\varepsilon\,\square$und der Belastung $\sigma$ umfassen.

4. Ultraschalldiagnosevorrichtung nach Anspruch 1, wobei die Kompressionszustand-Bewertungseinheit (19) konfiguriert ist, den Kompressionszustand basierend auf einer Beziehung der Verformungsänderung zu bewerten, die zwei Bezugsbereichen entspricht, die in zwei unterschiedlichen Geweben festgelegt wurden.

5. Ultraschalldiagnosevorrichtung nach Anspruch 4, wobei die Kompressionszustand-Bewertungseinheit (19) konfiguriert ist, den Kompressionszustand basierend auf einem Verhältnis der Verformungsänderung (= $\varepsilon1/\varepsilon2$ oder $\varepsilon2/\varepsilon1$) entsprechend den zwei Bezugsbereichen zu bewerten.

6. Ultraschalldiagnosevorrichtung nach Anspruch 4, wobei die Kompressionszustand-Bewertungseinheit (19) konfiguriert ist, den Kompressionszustand entsprechend dem Verhältnis der Verformungsänderung basierend auf einer Beziehung zwischen einem Verhältnis $\Delta\varepsilon2/\Delta\varepsilon1\,\square$der Verformungsänderungen in den zwei Geweben und des Kompressionszustands, der vorher gemessen und gespeichert wurde, zu erhalten.

7. Ultraschalldiagnosevorrichtung nach Anspruch 3, wobei die Elastizitätsbild-Erzeugungseinheit ein Bild der Belastung oder des Kompresssionszustands, das durch die Kompressionszustand-Bewertungseinheit (19) erhalten wurde, unter Verwendung wenigstens eines numerischen Werts, eines Histogramms oder eines Graphen erzeugt.

8. Ultraschalldiagnosevorrichtung nach einem der Ansprüche 4 bis 6, wobei das Gewebe, in dem der Bezugsbereich festgelegt wurde, wenigstens ein Muskel ist.

**Revendications**

1. Appareil de diagnostic à ultrasons comprenant :

une unité de calcul d'élasticité (13) pour calculer des données d'élasticité d'un tissu à une pluralité de points de mesure basés sur une paire de données de trame de signaux d'écho de réflexion mesurés chacun à un temps différent, qui sont obtenus dans un processus dans lequel un état de compression d'un objet est changé ; une unité de génération d'image d'élasticité pour générer une image d'élasticité basée sur les données d'élasticité calculées par l'unité de calcul d'élasticité de manière à être affichée sur l'unité d'affichage (10) ; et une unité d'évaluation d'état de compression (19) pour évaluer l'état de compression basé sur des données de changement de distorsion du tissu au point de mesure, dans lequel l'état de compression évalué corrélé avec l'image d'élasticité est affiché sur l'unité d'affichage, dans lequel l'unité d'évaluation d'état de compression (19) est configurée pour évaluer l'état de compression sur la base d'une valeur cumulative ($= \sum \Delta \varepsilon$) du changement de distorsion du tissu au point de mesure, **caractérisé en ce que** l'unité d'évaluation d'état de compression (19) est configurée pour acquérir le changement de distorsion $\Delta \varepsilon$ des tissus au point de mesure dans une région de référence choisie dans un tissu spécifique, et pour obtenir la distorsion cumulative $\varepsilon$ ($= \sum \Delta \varepsilon$) du tissu spécifique sur la base du changement de distorsion $\Delta \varepsilon$, et une contrainte $\sigma$ correspondant à la distorsion cumulative $\varepsilon$ basée sur une caractéristique contrainte/distorsion mesurée et stockée du tissu spécifique de manière à évaluer l'état de compression sur la base de la contrainte obtenue $\sigma$.

2. Appareil de diagnostic à ultrasons selon la revendication 1, dans lequel l'unité d'évaluation d'état de compression (19) est configurée pour acquérir le changement de distorsion $\Delta \varepsilon$ du tissu dans une région cible fixée à partir de l'unité de calcul d'élasticité (13) et pour obtenir une distorsion cumulative $\varepsilon$ en accumulant des valeurs du changement de distorsion $\Delta \varepsilon$ depuis un état dans lequel l'état de compression est égal à zéro.

3. Appareil de diagnostic à ultrasons selon la revendication 1, dans lequel :

l'unité d'évaluation d'état de compression (19) est configurée pour délivrer la contrainte obtenue $\sigma$ à l'unité de calcul d'élasticité (13) ; et l'unité de calcul d'élasticité (13) est configurée pour obtenir les données d'élasticité incluant au moins un module d'élasticité par rapport à la région cible fixée sur la base du changement de distorsion $\Delta \varepsilon$ et de la contrainte $\sigma$.

4. Appareil de diagnostic à ultrasons selon la revendication 1, dans lequel l'unité d'évaluation d'état de compression (19) est configurée pour évaluer l'état de compression sur la base d'une relation du changement de distorsion correspondant à deux régions de référence fixées dans deux tissus différents.

5. Appareil de diagnostic à ultrasons selon la revendication 4, dans lequel l'unité d'évaluation d'état de compression (19) est configurée pour évaluer l'état de compression sur la base d'un rapport du changement de distorsion ($= \varepsilon 1/\varepsilon 2$ ou $\varepsilon 2/\varepsilon 1$) correspondant aux deux régions de référence.

6. Appareil de diagnostic à ultrasons selon la revendication 4, dans lequel l'unité d'évaluation d'état de compression (19) est configurée pour obtenir l'état de compression correspondant au rapport du changement de distorsion basé sur une relation entre un rapport $\Delta \varepsilon 2/\Delta \varepsilon 1$ des changements de distorsion dans les deux tissus et l'état de compression, qui est mesuré de manière préliminaire et stocké.

7. Appareil de diagnostic à ultrasons selon la revendication 3, dans lequel l'unité de génération d'images d'élasticité génère une image des contraintes ou l'état de compression obtenu par l'unité d'évaluation d'état de compression (19) en utilisant au moins un moyen parmi une valeur numérique, un diagramme à barres, et un graphique.

8. Appareil de diagnostic à ultrasons selon l'une quelconque des revendications 4 à 6, dans lequel le tissu ayant la région de référence choisie dans celui-ci est au moins un muscle.

FIG.1

FIG.2

FIG.3

## FIG.4

SET ROI — S1

INITIALIZE $\Sigma\Delta\varepsilon ij$ OF $\Delta\varepsilon ij$ VALUES WHILE CONTACTING PROBE WITH OBJECT IN NONPRESSURE STATE — S2

MEASURE ULTRASONIC WAVE WHILE APPLYING PRESSURE TO OBJECT — S3

CALCULATE $\Delta\varepsilon ij$ AT Pij IN PREDETEMRINED REGION INCLUDING ROI — S4

CALCULATE $\Sigma\Delta\varepsilon$ OF $\Delta\varepsilon$ VALUES IN ROI — S5

GENERATE ELASTICITY IMAGE BASED ON $\Delta\varepsilon ij$ AND DISPLAY $\Sigma\Delta\varepsilon$ IN CORRELATION WITH ROI — S6

EP 2 030 572 B1

FIG.5

FIG.6

FIG.7

$$\sigma = \exp(\alpha 1 \cdot \varepsilon 1)$$

FAT

STRESS $\sigma$ [Pa]

$\sigma$

$\Delta \sigma$

$\Delta \varepsilon 1$

0

$\varepsilon 1$

DISTORTION $\varepsilon 1$[%]

EP 2 030 572 B1

FIG.8

```
                          ▽

   ┌─────────────────────────────────────────────┐
   │            SET ROI, R1 AND R2               │ ⟵── S11
   └─────────────────────────────────────────────┘

   ┌─────────────────────────────────────────────┐
   │ MEASURE ULTRASONIC WAVE BY CONTACTING PROBE │ ⟵── S12
   │ WITH OBJECT WHILE APPLYING PRESSURE TO OBJECT│
   └─────────────────────────────────────────────┘

   ┌─────────────────────────────────────────────┐
   │ CALCULATE Δεij AT Pij IN PREDETERMINED REGION│ ⟵── S13
   │               INCLUDING ROI                  │
   └─────────────────────────────────────────────┘

   ┌─────────────────────────────────────────────┐
   │ CALCULATE RATIO Δε2/Δε1 OF Δε1 AND Δε2 IN R1 and R2 │ ⟵── S14
   └─────────────────────────────────────────────┘

   ┌─────────────────────────────────────────────┐
   │     CORRELATE Δεij WITH Δε2/Δε1 TO GENERATE  │ ⟵── S15
   │  ELASTICITY IMAGE TO BE DISPLAYED REAL-TIME  │
   └─────────────────────────────────────────────┘

                          ▽
```

# FIG.9

EP 2 030 572 B1

FIG.10

STRESS σ [Pa]

$\sigma = \exp(\alpha 2 \cdot \varepsilon)$

$\sigma = \exp(\alpha \cdot \varepsilon)$

MUSCLE

TARGET PORTION

$\sigma = \exp(\alpha 1 \cdot \varepsilon)$

$\Delta \varepsilon 2$

$\Delta \varepsilon 1$

$\Delta \sigma$

$\Delta \sigma$

σ

FAT

0

DISTORTION $\varepsilon$ [%]

EP 2 030 572 B1

FIG.11

EP 2 030 572 B1

# FIG.12

$\sigma = \exp(\alpha 1 \cdot \varepsilon)$

NON-LINEARITY $\alpha 1'$

NON-LINEARITY $\alpha 1$

STRESS $\sigma$ [Pa]

28

$\sigma f$

$\varepsilon f$

DISTORTION LIMIT

DISTORTION $\varepsilon$ [%]

0

COMPRESSION LIMIT REGION

EP 2 030 572 B1

FIG.13

# FIG.14

EP 2 030 572 B1

## FIG.15

SET L-ROI INCLUDING ROI, R1 AND R2 —— S21

MEASURE ULTRASONIC WAVE BY CONTACTING PROBE TO OBJECT WHILE APPLYING PRESSURE TO OBJECT —— S22

CALCULATE $\Delta\varepsilon ij$ AT Pij in L-ROI —— S23

CALCULATE $\Delta\varepsilon$mean OF $\Delta\varepsilon$ VALUES IN L-ROI —— S24

CALCULATE $\Delta\varepsilon ij/\Delta\varepsilon$mean THROUGH NORMALIZATION BY DIVIDING $\Delta\varepsilon ij$ AT Pij BY $\Delta\varepsilon$mean —— S25

GENERATE AND DISPLAY NORMALIZED DISTORTION IMAGE BASED ON $\Delta\varepsilon ij/\Delta\varepsilon$mean —— S26

# FIG.16

EP 2 030 572 B1

FIG.17

$$\frac{Ntot/\alpha 1}{N1/\alpha 1 + N2/\alpha 2 + N/\alpha}$$

NORMALIZED DISTORTION CHANGE $\Delta \varepsilon_{norm} 1$

$\Delta \varepsilon_{norm} 1(t)$

0    $\sigma(t)$   $\sigma f$    STRESS LIMIT    STRESS $\sigma$ [Pa]

(A)

$$\frac{Ntot/\alpha 2}{N2/\alpha 2 + N/\alpha}$$

NORMALIZED DISTORTION CHANGE $\Delta \varepsilon_{norm} 2$

$\Delta \varepsilon_{norm} 2(t)$

0    $\sigma(t)$   $\sigma f$    STRESS LIMIT    STRESS $\sigma$ [Pa]

(B)

EP 2 030 572 B1

FIG.18

(A)

(B)

EP 2 030 572 B1

FIG.19

(A)

(B)

EP 2 030 572 B1

## FIG.20

(A)

(B)

EP 2 030 572 B1

## FIG.21

(A) STRESS $\sigma < \sigma f$

NORMALIZED DISTORTION CHANGE $\Delta \varepsilon_{norm}$

NEGATIVE GRADIENT OF LINEAR APPROXIMATION

FAT | TARGET PORTION | MUSCLE | DEPTH DIRECTION [mm]

(B) STRESS $\sigma > \sigma f$

NORMALIZED DISTORTION CHANGE $\Delta \varepsilon_{norm}$

POSITIVE GRADIENT OF LINEAR APPROXIMATION

FAT | TARGET PORTION | MUSCLE | DEPTH DIRECTION [mm]

EP 2 030 572 B1

# FIG.22

R1 (1, 2, ...L)

1  2  3  4  ...  M

35

FAT

TARGET
PORTION

ROI(1, 2, ...L)

1  2  3  4  ...  M

MUSCLE

1  2  3  4  ...  M

R2 (1, 2, ···L)

100
80
60
40
20
0

EP 2 030 572 B1

## FIG.23

EP 2 030 572 B1

FIG.24

MAMMARY GLAND

EXTRACTION OF FAT

FAT

MAMMARY GLAND

MUSCLE

B MODE IMAGE

(A)

FAT

MUSCLE

EXTRACTION OF MUSCLE

(B)

FIG.25

CLICK TO SELECT

MAMMARY
GLAND

EXTRACT THE SAME
TISSUE REGION AS
CLICKED REGION

FAT

MAMMARY
GLAND

MUSCLE

B MODE IMAGE

(A)

FAT

MUSCLE

EXTRACTION OF MUSCLE

(B)

FIG.26A

FIG.26B

## FIG.26C

BEFORE COMPRESSION

TIME t-1

B MODE IMAGE

y1(t-1)

x1(t-1)

x2(t-1)

(A)

AFTER COMPRESSION

TIME t

y1(t)

x1(t)

x2(t)

d( x2(t-1) )

(B)

DISPLACEMENT
DISTRIBUTION
BEFORE/AFTER
COMPRESSION

0    d( x(t-1) )

x1(t-1)

x2(t-1)

x(t-1)

(C)

EP 2 030 572 B1

# FIG.26D

BEFORE COMPRESSION

TIME t-1

AFTER COMPRESSION

TIME t

(A)

(B)

FIG.27

EP 2 030 572 B1

FIG.28

EP 2 030 572 B1

## FIG.29

σ-ε ZONE INDICATING HIGH POSSIBILITY OF DUCTAL BREAST CARCINOMA

47

48

STRESS σ [Pa]

σ-ε ZONE INDICATING HIGH POSSIBILITY OF FIBROUS ADENOMA

49

0

DISTORTION ε [%]

EP 2 030 572 B1

FIG.30

INTRADUCTAL PAPILLOMA (BENIGN)

RELATIONSHIP BETWEEN ELASTICITY INDEX AND STRESS INDEX OF BENIGN TISSUE

(A)

MADIDANS DUCTAL BREAST CARCINOMA (MALIGNANCY)

RELATIONSHIP BETWEEN ELASTICITY INDEX AND STRESS INDEX OF MALIGNANCY TISSUE

(B)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005120358 A1
- JP 2004261198 A
- JP 2005066041 A
- JP 2005118152 A
- WO 2005025425 A
- WO 2004039262 A

### Non-patent literature cited in the description

- **KROUSKOP T et al.** Elastic Moduli of Breast and Prostate Tissues Under Compression. *Ultrasonic Imaging,* 1998, vol. 20, 260-274